(19) Europäisches Patentamt / European Patent Office / Office européen des brevets

(11) **EP 4 350 002 A1**

(12) **EUROPEAN PATENT APPLICATION**

(43) Date of publication:
**10.04.2024   Bulletin 2024/15**

(21) Application number: **22200403.8**

(22) Date of filing: **07.10.2022**

(51) International Patent Classification (IPC):
**C12Q 1/6823** (2018.01)      **C12Q 1/6827** (2018.01)
**C12Q 1/6851** (2018.01)

(52) Cooperative Patent Classification (CPC):
(C-Sets available)
**C12Q 1/6823; C12Q 1/6827; C12Q 1/6851**   (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Biotype GmbH**
**01109 Dresden (DE)**

(72) Inventors:
• **Preussler, Matthias**
**01109 Dresden (DE)**

• **Weber, Anke**
**01109 Dresden (DE)**

(74) Representative: **Heide, Anna Katharina**
**Ruhr-IP Patentanwälte**
**Brucker Holt 58**
**45133 Essen (DE)**

Remarks:
The complete document including Reference Table(s) and the Sequence Listing(s) can be downloaded from the EPO website

(54) **NACHWEIS VON MOLEKULAREN ANALYTEN AUF DER GRUNDLAGE MASSGESCHNEIDERTER SONDENKONKURRENZ**

(57)     The invention provides a method for detection of at least one molecular genetic analyte comprising a upstream and a competitive downstream oligonucleotide probe, wherein the upstream probe has a sequence region (1) and the downstream probe has a sequence region (3), both have an overlapping region (2). The regions (1), (2) and (3) have similar melting temperatures (Tm) and wherein the downstream probe hybridizes with a decreasing hybridization rate in relation to the upstream probe to the target sequence with at least one analyte, and the upstream probe hybridizes with an increased hybridization rate in relation to the downstream probe to the target sequence with the at least one analyte. The Detection is based on the released hydrolysis product(s) from the respective probe and optionally in combination with the obtained amplified products. Additionally, the present invention provides a combination of probes and a Kit for use in the method.

Fig. 1

EP 4 350 002 A1

(52) Cooperative Patent Classification (CPC): (Cont.)

C-Sets
**C12Q 1/6823, C12Q 2525/186, C12Q 2531/113,
C12Q 2535/131, C12Q 2537/143,
C12Q 2561/101, C12Q 2565/102,
C12Q 2565/137;
C12Q 1/6827, C12Q 2525/186, C12Q 2531/113,
C12Q 2535/131, C12Q 2537/143,
C12Q 2561/101, C12Q 2565/102,
C12Q 2565/137;
C12Q 1/6851, C12Q 2525/186, C12Q 2535/131,
C12Q 2537/143, C12Q 2561/101,
C12Q 2565/102, C12Q 2565/137**

**Description**

This invention generally relates to the field of nucleic acid chemistry, specifically methods for detection and quantification of nucleic acids and more specifically the field of in vitro molecular diagnostics.

The invention provides a method for detection of at least one molecular genetic analyte comprising a upstream and a competitive downstream oligonucleotide probe, wherein the upstream probe has a sequence region (1) and the downstream probe has a sequence region (3), both have an overlapping region (2). The regions (1), (2) and (3) have similar melting temperatures (Tm) and wherein the downstream probe hybridizes with a decreasing hybridization rate in relation to the upstream probe to the target sequence with at least one analyte, and the upstream probe hybridizes with an increased hybridization rate in relation to the downstream probe to the target sequence with the at least one analyte. The Detection is based on the released hydrolysis product(s) from the respective probe and optionally in combination with the obtained amplified products. Additionally, the present invention provides a combination of probes and a Kit for use in the method.

Molecular diagnostics of nucleic acids is a collection of techniques used to analyse qualitative or quantitative nucleic acid sequence variants (see also analyte and biomarker definitions in this patent application for more details). These variants can be now described in comparison to reference sequences which are currently established, validated with statistically significant sample sizes, and stored in curated databases. For example, in human diagnosis more than 1000 wildtype or healthy individuals are compared to patients with germline or somatic mutations for which a correlation to a physiological state or disease is well documented.

To analyse these variants several prior art technologies have been established. Standard qualitative polymerase chain reaction (PCR) and quantitative PCR (qPCR) are well known to experts in this field. They can also be combined with a reverse transcription step to genotype and/or quantify RNA. The qPCR technologies can be performed in uniform assays in combination with in situ fluorescent optical read out instruments (e.g. qPCR thermocycler) using a dsDNA selective fluorescent dye (like SYBR™ Green) or FRET (Foerster Resonance Energy Transfer) probes which bind specifically to the amplified target DNA. FRET probes like Molecular Scorpions™ or Molecular Beacons bind reversibly to the amplified target DNA whereas hydrolysis probes (sometimes also referred to as TaqMan™ probes, a trademark of Roche Diagnostics International AG, Rotkreuz, CH) are hydrolysed after each PCR cycle by the intrinsic nuclease of Taq DNA polymerase. Allele specific FRET probes can also be combined with different fluorescent colours to distinguish, in some extend, SNPs or deletion-insertion polymorphisms (DIPs) in one single qPCR. The use of FRET probes offers in addition a confirmation step for the amplified target DNA which is sometimes stipulated as a quality standard by analytical or medical guidelines.

Multi-parameter or multiplex technologies for nucleic acid amplification and genotyping or quantification are preferable for several analytical and differential diagnostic applications. Syndromic disease which shows the same or similar symptoms like infections (virus, microbes and parasites) of the same organs (e.g. lung, urogenital tract, skin) or inheritable diseases like cranial dysmorphisms or conspicuousness of behaviour are such examples. Other examples are cancer stratification for individual therapy or forensic and paternity testing for the latter of which a high discrimination power to distinguish individuals of a species is necessary. These can be only achieved by combining 12 - 24 multiallelic short tandem repeats (STRs) or more than 30 biallelic analytical markers like SNPs or DIPs (syn. InDels). Technologies which allow these degrees of multiplexing are for example cartridge devises (e.g. Film Array System, bioMérieux, Marcy-l'Étoile, FR; Vivalytic, Bosch Healthcare Solutions GmbH, Waiblingen, DE) which are point of need instruments that integrate all steps from sample preparation to read out. The core technology of these devices is called geometric multiplexing which means that individual PCR or qPCR are performed in separate cavities of the cartridge. Other approaches of geometric multiplexing PCR are known as digital droplet (or emulsion) PCR (ddPCR) (e.g. QX200 Droplet Digital PCR System, Bio-Rad Laboratories Inc., Hercules, US-CA) or Next Generation Sequencing (NGS) instruments which also use emulsion PCR in combination with distinct nanoliter cavities and solid beeds (Iron Torrent System, Thermo Fisher Scientific Inc., Waltham, Massachusetts, USA) or DNA chips with discrete areas (Illumina Inc., San Diego, US-CA) to separate individual DNA targets for amplification and genotyping. NGS technologies can also be used for absolute quantification with high multiplexing capability. Another approach of multiplexing which is well accepted standard in forensic sciences or paternity testing combines a one-step endpoint multiplex PCR with capillary electrophoresis (CE) to separate amplicons by different length and/or fluorescent dyes. This technology is more sensitive and faster to address the needs of the end-users in this field. In these tests one PCR primer of a primer pair in the multiplex PCR is labelled by a fluorescent dye which is covalently bond to the 5'-end. Afterward, the reaction mixture is injected into a CE device for qualitative or semi quantitative analyses. More than 25 STRs or DIPs can be genotyped or even DNA mixtures in forensic stains of up to three offenders can be distinguished. The Modaplex device (see definitions of this patent application) which was used in this document combines a multiplex qPCR approach with a CE to achieve the genotyping and quantification of up to 50 parameters in one reaction setting.

Limits of standard qPCR are that the degree of multiplexing for genotyping and quantification by FRET probes is up to 6 diagnostic parameters. This degree is even reduced if internal amplification controls must be included as quality standards according to analytical or medical guidelines (e.g. design of in vitro diagnostic devices). In addition, some relative quantification protocols need parallel reactions as reference standard which must be additionally performed. NGS and ddPCR are very expensive, need high amounts of sample nucleic acid of high quality, and multistep protocols like DNA libraries construction in case of NGS which are time consuming with a set of automation steps including validated bioinformatics for quality selection of primary data and sequence annotation to guarantee reproducibility and safety. Cartridge devices also possess drawbacks in assay development and production which reduces the flexibility for assay design which are especially of need in biomarker development and clinical validation studies. Another important challenge of standard qPCR technologies consists in the detection and quantification of minor DNA variants in a mixture with an excess of closely related DNA copies. Examples for this are somatic cancer mutations which must be distinguished from wildtype DNA in surgical or liquid biopsies, and the detection of small amounts of fetal DNA in the blood circulation of pregnant women to determine aneuploidies and other chromosomal abnormalities (e.g. segmental copy number variations) in case of non-invasive prenatal testing (NIPT). Furthermore, genetic polymorphisms are frequently used for chimerism analysis after allogeneic human blood stem cell transplantation to early monitor minimal residual diseases (relapse of leukaemia) or to detect within forensic stains traces of offender DNA in complex mixtures with victim derived nucleic acid.

Considering the above, there are different technologies wherein a competition or blocking event is used to manipulate hybridization of oligonucleotide to a nucleic acid sequences within dPCR, ddPCR or qPCR or additional pre-enrichment steps before NGS library preparation. Those prior art PCR methods consist mostly of two competing forward primer and one reverse primer for amplification. These so called "Blocker Displacement Amplification" technologies, e.g. primer-blocker-system, have the aim to enrich minor variants like SNPs for next-generation sequencing and block amplification of the wildtype sequence.

Technologies applying probes and primers in competition for digital droplet PCR (ddPCR) are known, wherein a two primer and two probes approach uses the competition of two probes. The readout is described as one signal for either mutant or wildtype. In general, the dPCR and ddPCR focused applications constitute an endpoint PCR analysis. The accumulated probe signals at the end of a PCR reaction are measured in each partition (a.k.a. droplet or planar area on a chip) and the readout enables discrimination between positive and negative signals only. Such methods usually further require sequencing of the specifically enriched mutated sequence or additional variant specific probes for discrimination. Thus, the design of such assays is more complex, time consuming, cost intensive, troublesome and require high professional experts. Additionally, from the diagnostic point of view the skilled person has to await the end of PCR and to apply a further method to achieve a result and a final diagnosis.

Compared thereto the method of the present invention, does not rely on the enrichment of the mutant (analyte with variation), based on one of the primers, while another oligonucleotide, such as another primer or probe, blocks the amplification on the wildtype sequence. In contrast, in the method of the present invention a non-discriminatory amplification of the target region without discrimination of wildtype or mutations in the sequence is performed. The method of the present invention utilizes two competing probes for discrimination of either wildtype or mutated status via a parallel signal increase/decrease (signal exchange) that can be measured by means of an end-point PCR or is instantly (continuously) measured by a real-time PCR, preferably by means of the Modaplex technology (Biotype GmbH, Dresden). Compared to the prior art, the method of the present invention does not block the amplification. Herein, it is described for the first time to use a pair of two probes, which are adjusted to each other as described herein for the detection of certain polymorphisms in qPCR, in a qPCR with CE, preferably in a Modaplex device.

The advantages of the present invention are, that the method also allows for real-time analysis of both probe signals (e.g. for the wildtype and a variation, e.g. a nucleotide polymorphisms) during the ongoing PCR reaction in relatively quantitative manner. Such CE devices, preferably Modaplex, perform measurements which include cycle threshold values (Ct or Cp = "crossing point", as defined as cycle number at detection threshold according to Roche Diagnostics instruments) and RFU. Based on the capability of the CE technology to measure both probe signals in one color channel, both signals can be directly compared with each other which enables various evaluations, e. g. the ratio of both signals. The aforementioned comparison allows for to quantify the allelic ratio of polymorphisms on DNA or RNA level. This direct comparison of signals is limited for common dPCR, ddPCR or qPCR instruments as signals must be acquired separately in different color channels or even in different reactions on the same plate. Comparison of separated signals in dPCR, ddPCR and qPCR are biased by color channel differences (for example unequal signal strengths) and reaction mix deviations in well-to-well comparisons.

Compared thereto, the present invention provides a probe and method for real-time analysis, (also in uniform reactions comprising several copies of the at least one target) which allows simultaneous detection of a plurality of variants with one probe combination according to the present invention but also a probe and method for digital PCR technologies using partitions as well as a universal probe designed feasible for both mentioned methods. The unique CE technology, preferably Modaplex, additionally enables the discrimination of SNP and Insertion/Deletion (InDel) mutations. It is worth mentioning that according to the method and probes of the present invention even SNPs and 1 bp DIPs are detectable, and in particular complex mutation hotspots (or mutation island). Labeling one of the primers (forward or reverse), the main amplicon, in particular the mutant sequences (A1-v, explained below) can be detected by gel-migration length on the Modaplex. The Migration is correlated with the amplicon size. InDel mutations would cause an altered migration length which would be detected. Using the CE technology, preferably Modaplex, facilitates the multiplexing of at least two hydrolysis product (F1-up/F1-downs) detections in one reaction without impeding the measurement sensitivity or reliability. At the same time, more PCR reactions may be performed in the same multiplex using defined labels that can be differentiated from the at least two hydrolysis products (F1-up/F1-downs). In contrast, multiplexing is limited with the dPCR or ddPCR technology due to the limited amount of color channels.

Further, so fare no prior art teaching provides guidelines for the design of competing probes as described herein based on the melting temperature of the respective probes or their free enthalpy. The probes of the present invention and the method therefore are novel and non-obvious in view of the prior art and offers precise and fast molecular diagnostics for both, end-point and real time PCR, in particular for (relative) quantification of genetic variants. While prior art technologies and workflows require several business days, the method of the present invention, preferably on a CE-device, such as Modaplex, delivers results in hours.

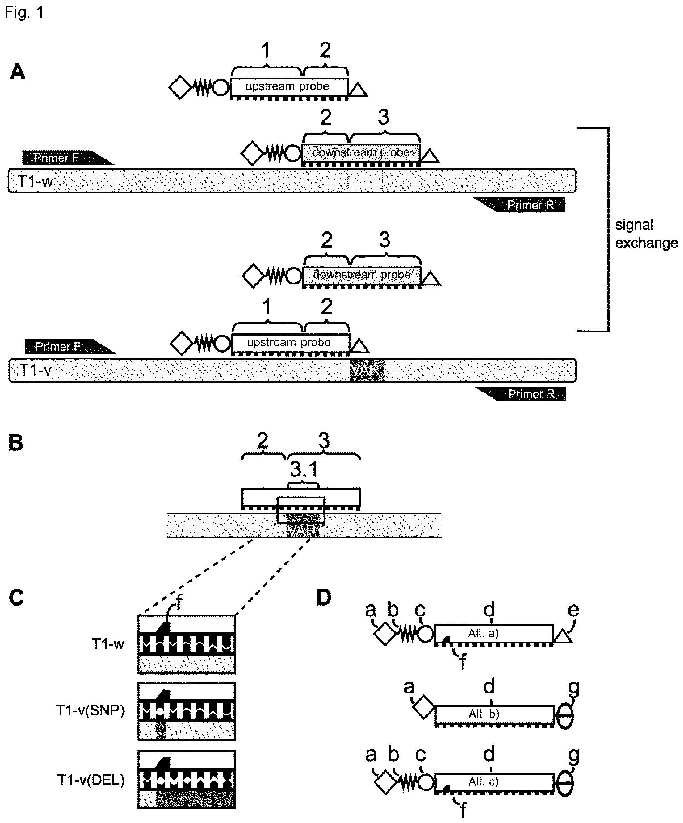

In more detail the technology of the present invention utilizes two primers (F, R) and two probes (downstream and upstream), combining multiple PCR based analysis methods in one approach to detect hotspot mutations. The design enables detection of several different mutations (defined herein as variation) that can occur in the same genetic region or hotspot. An example of such a region can be a several nucleotides spanning hotspot, a codon or a genetic polymorphism (VAR, see Fig. 1). Experiments using the method and probes of the present invention show SNP detection of 6 to 8 different SNP in three base pair spanning regions, insertion detection of up to 4 base pairs and deletion detection of up to nine base pairs. In consequence, one can expect every sequence variability causing a mismatch between competitive downstream probe and target sequence, to be detectable. Furthermore, decisions for therapy are more and more based on mutation status with recommendations and classifications in the WHO cancer guidelines. The present invention (probes and method) enables fast analysis of mutation status giving a yes/no reply, which is sufficient for most clinical decisions. This means, that the pending therapy decision is independent of knowing the exact genetic sequence but rather a reliable detection of any mutation in a specific hotspot. In this respect the additional advantage is being able to detect also (thus far) unknown variants if the variant occurs in the same hotspot region. The application of long-term molecular diagnostics (i.e. to identify the exact genetic variant) based on sequencing approaches can be postponed or omitted. For fast decision, e.g. in cancer diagnostics, the technology enables molecular status analysis in a time frame of 4 hours.

**The advantages of the present invention** in particular are

- The skilled person can make use of the Taq_Man ™ probe design and adapt it according to the present invention as described herein.
- the same probes, in particular the same probes combination, are feasible for various mutations and most particular for mutation hotspots
- no specific adaptation of the probe to the mutant sequence is necessary which saves time and material (economic aspect),
- the basic probe design of the present invention is feasible for both, digital PCR technologies including NGS and real-time PCR (flexibility aspect)
- SNP and one bp mutation are detectable and distinguishable from the wildtype signal (specificity).
- Detection of any mutation in real-time is enabled (range of application)
- a time saving, fast result on degree of variation and type of mutation is provided,
- high sensitivity for low degree of variant even for SNP and one bp deletions,
- a high multiplexing grade is possible and
- a fully automated detection method is possible (easy handling for less experienced staff)
- an internal amplification control indicating the presence of the analyte within the test sample to avoid false negative results.

The method of the present invention is designed to overcome the disadvantages from the above-mentioned prior art and to provide an improved method and oligonucleotide probes to detect, identify, separate and quantify multiple genetic variants in one PCR-based reaction with a desired amount (multiplex degree) of different genetic analytes (within target sequences) in a sample. It is an object of the present invention to reduce the count of required primers or probes to detect multiple variants of different hot spot regions in one multiplex PCR reaction, leading to enhanced multiplex robustness and enabling mutational status output for multiple variants. This is especially important for diagnostic purposes for example in molecular tumor diagnostics which use molecular tumor characterisation for therapy decisions (e.g., glioma or AML). Dependent on this, it is an object to provide fast molecular stratification by a multiplex PCR, end-point or real-time PCR, approach that is conducted in hours rather than days as comparable technologies like NGS. Thus, another object of the present invention is to provide a time saving, material saving and consequently an economic PCR approach for reliable detection of genetic analytes containing a plurality of different variations in a specified region (hot spot). Another object of the invention is to provide a method and oligonucleotide probes for use in the method, wherein the required oligonucleotide probes are designable based on the target sequence without variation (T1-w), as target sequences without variation are commonly accessible in several free available databases (e.g., UCSC Genome Browser, NCBI Blast, Ensembl genome browser). Thus, it is an object to provide a method to detect, identify, separate, and quantify any known and putative genetic polymorphism in an analyte. Another object is to provide a method, for singleplex and any multiplex degree, of high sensitivity for genetic polymorphism detection on the different PCR platforms, such as real time PCR and digital PCR and in particular for real time PCR in combination with a CE (such as Modaplex device). For that the equilibrium-based approach for upstream and downstream probe as described herein enables high sensitivity to any shift in the template concentrations in a mixed template PCR reaction with analytes with or without variation. It is another object to provide an accurate measurand for the degree of variation that can be important for diagnostic appraisals such as early detection of minimal residual disease (MRD) during efficacy monitoring under therapy or prior adjustment of individual therapy. Therefore, the signal shift of the two probe hydrolysis products (F1-up/F1-Down) can be used to calculate ratios of two competing alleles within a broad linear measuring range, in particular in a digital PCR and real-time PCR. Therefore, it is another object of the present invention to provide an universal probe design for the detection of variants which is feasible for and can be applied on well-known PCR devices, as described herein. Furthermore, it is an object to provide a practicable design for appropriate oligonucleotide probes and tailored for the specified nucleic acid amplification and genotyping method. However, as the application of well-known software applications for oligo design do not allow appropriate calculation of competing probes, it is the object of the present invention to provide sufficient guidance to create feasible probes by means of numerous accessible software applications (e.g., Tm Calculator [ThermoFisher, USA], Geneious Prime [Geneious, New Zealand], OligoAnalyzer™ Tool by Integrated DNA Technologies [IDT, USA]). However, calculating the competition of at least two probes as in the herein described method is impossible with prior art applications by means of software. Thus, the herein described method provides innovative design rules for competing probes.

Thus, the clear and distinct detection of different hydrolysis products of the probes representing different analytes is achieved by the appropriate combination of modifications according to the invention. Therefore, it is another object of the invention to provide different probe combinations and modifications to enable a multiplex approach for the detection of different analytes by different migration behaviour of their respective hydrolysis products in a capillary electrophoresis without unwanted artefacts. Thus, another object of the invention is to provide a method enabling different migration behaviour of the released hydrolysis products for a desired number of analytes and different single nucleotide polymorphisms in a sample. It is further an object to provide a method for detecting and quantifying the desired number of hydrolysis products of said probe combinations that are generated due to a 5' to 3' nuclease activity of a nucleic acid polymerase or a mixture of enzymes with the same but separate activities. For that a detectably labelled oligonucleotide probe, in particular of alternative a) and/or c), is provided that is cleaved or released due to a 5' to 3' nuclease activity and a nucleic acid polymerase activity after hybridization to its respective target sequence. Finally, it is an object to provide a method as described above that is full automated and can be performed in a closed and cross-contamination free system like any known capillary electrophoreses (CE) devices such as the Modaplex device or other systems of Thermo Fisher Scientific Inc., Promega Corp, Fullerton, Qiagen GmbH, Beckman Coulter, Syntol, Agilent Technologies Inc. and other.

The method is designed to overcome the disadvantages from the above mentioned prior art and to provide an improved method and oligonucleotide probes to detect, identify, separate and quantify multiple genetic variants in one PCR-based reaction with a desired amount (multiplex degree) of different genetic analytes (within target sequences) in a sample.

The novel and unified inventive solution for all aspects of the present invention as described herein, is realized and controlled by appropriate adjustment of the melting temperatures of all oligos and primary by the ΔTm of the competing probes in relation to the wildtype sequence (in particular region (3) in relation to T1-w). The probe design relays on said ΔTm, the hybridization behaviour of said probes in the method relays on said ΔTm, consequently the release of any detectable signals depend on the hybridization driven by said ΔTm. The creation of an appropriate probe combination relays on said ΔTm and the components of the kit as well. Finally, the underlying unified inventive approach for all aspects (and all embodiments) is a probe combination of an adjusted ΔTm (in particular region (3) and/or region (1)) in relation to their respective complementary sequence on the wildtype target (T1-w).

In conclusion, the method and the probes of the present invention will be of utmost importance for the market of molecular tumor diagnostics. The detection of variations (e.g. Fig. 1 C) by means of the presented technology prior to cost intensive NGS diagnostics is already substantial for molecular tumor diagnostics and is expected to be essential for various upcoming assays.

The inventive combination probes and method is useful for the specific detection and quantification of an individual analyte within a target sequence in a sample, but also provide multiplex application for the detection and quantification of multiple analytes in a sample. The inventive subject matters are defined in the claims. The inventive concept is described in the following by the described aspects as well as preferred embodiments without being restricted thereto. Each of the described aspects can be combined with each other, without explicitly describing any individual embodiment. Examples are showing the feasibility of the invention.

The first aspect is a method for the detection of at least one or more molecular genetic analytes comprising

- providing a reaction mixture comprising

  • at least one molecular genetic analyte comprising at least one target sequence (T1) without a variation (T1-w) and potentially at least one molecular genetic analyte comprising at least one target sequence with at least one variation (T1-v),
  • a primer pair (F, R) flanking the at least one T1-w and the at least one T1-v being suitable for non-discriminatory amplification of the target sequences,
  • at least one upstream oligonucleotide probe ("upstream probe") comprising

    (i) a sequence region (1) and a sequence region (2), which together compose a sequence which is complementary to a sequence that is the same on the target sequence without a variation (T1-w) and on the target sequence with at least one variation (T1-v), preferably region (1) and region (2) respectively have a sequence length of at least 5 nt up to 20 nt, and

  • at least one competitive downstream oligonucleotide probe ("downstream probe") comprising

    (i) a sequence region (3) and a sequence region (2), which together compose a sequence which is complementary to a sequence on the target sequence without a variation (T1-w), preferably region (3) and region (2) respectively have a sequence length of at least 5 nt up to 20 nt, and
    (ii) at least one mismatch within the region (3) in relation to the at least one variation on the target sequence (T1-v),

  • the 3'-end of the upstream and 5'-end of the downstream probe are complementary to the partially overlapping region (2) on a sequence 3' upstream of the at least one variation on the at least one target sequence (T1-v), and
  • the sequence region (1) and the sequence region (3) have a similar melting temperature (Tm) and a $\Delta$ Tm sequence region (1) to Tm sequence region (3) of less than or equal to 6°C,
    wherein **each** probe further comprises:

    (iii) a protective group (3'-blocker) at the 3'-end of probe inhibiting a primer extension reaction, and
    (iv) at least one cleavable hydrolysis product from the 5'-end of the respective probe (F1-Down and/or F1-up) comprising at least one nucleotide of the 5'-end of the respective probe, and

  • the at least one downstream probe and/or the upstream probe comprises (v) a label at 5'-end,

the method comprising the steps
- optionally provision of a sample comprising at least one target sequence without a variation (T1-w) and/or of a sample comprising at least one target sequence with at least one variation (T1-v),
- optionally preparation of the sample(s) and isolation of nucleic acid material comprising at least one target sequence without a variation (T1-w) and/or at least one target sequence with at least one variation (T1-v), respectively,
- preferably providing a reaction mixture comprising the above probe combination, primer and all further components to perform the method accordingly,
- contacting the at least one target sequence, in particular at least one target T1-v and/or T1-w, with the at least one downstream probe and the least one upstream probe and the at least one primer pair (F, R), in particular with the outflanking primer pair (F, R) suitable for amplifying the target sequences,
- hybridization of the at least one primer pair (F, R) in particular to the respective sequence on the at least one target sequence and at least one primer hybridizes to a different strand of the at least one target sequence,
- preferably non-discriminatory amplification of the respective sequences by the at least one primer pair (F, R),
- hybridization of at least one probe to its respective complementary sequence on the at least one target sequence, wherein the respective probe hybridizes within the region flanked by the primer pair (F,R), preferably both probes, preferably of the respective combination of probes a), b) or c), hybridizes, and

  • the upstream probe and downstream probe hybridize in equilibrium to the at least one target sequence without a variation (T1-w),

- the downstream probe hybridizes with a decreasing hybridization rate in relation to the upstream probe to the, preferably potentially, at least one target sequence with the at least one variation (T1-v), and
  - the upstream probe hybridizes with an increased hybridization rate in relation to the downstream probe to the at least one target sequence with the at least one variation (T1-v),

- cleavage of the hybridized probe(s) with a target -dependent 5' to 3' nuclease activity to release the at least one cleavable hydrolysis product (F1-Up and/or F1-Down),
- optionally separation of the released hydrolysis product (F1-Up and/or F1-Down),
- optionally separation of the obtained amplified sequence T1-w and/or T-1v, in particular of the respective amplicons (amplificate synonym) A1-w and/or A1-v, and
- detection of the achieved hydrolysis product(s) (F1-Up and/or F1-Down).

The aforementioned applies for each probe combination a), b) and c), respectively, as described herein.

If the at least one target sequence with at least one variation (T1-v) is not present, the method according to the present invention detects two signals representing T1-w. That could be the case, if the provided sample therefrom nucleic acid material is isolated for the method according to the present invention, lacks appropriate material isolated from e.g. blood, tissue, liquid or solid biopsy material. Another reason is, that the patient does not carry any variation and therefore no variation can be detected. In both cases both probes, the downstream and upstream probe of the present invention, will hybridize to the complementary sequence and two signals are obtained within the method according to the present invention. Furthermore, it is advantageous to always obtain at least one internal amplification/reaction control by the detection of a signal (F1-up/F1-down) in samples containing analytes with (T1-v) as well as without variation (T1-w). If desired the amplicons (A1-w/A1-v) may be detected as additional control by use of labeled primers.

In a second aspect the method of the present invention, with increasing $\Delta$Tm of Tm sequence region (3) to Tm of the respective sequence on T1-v comprising the at least one analyte, the hybridization rate of the downstream probe to T1-v decreases. In particular the hybridization rate of the downstream probe to the respective complementary sequence of T1-v decreases. Preferably, region (1), region (2) and region (3), respectively have a sequence length of at least 5 nucleotides (nt) up to 25 nt, more preferably up to 20 nt. More preferably each region has a length of at least 5 nucleotides (nt) up to 10 nt. In particular, the respective sequence length may be 5, 6, 7, 8, 9, 10, 11, 12, 13, 14, 15, 16, 17, 18, 19, 20, 21 ,22 ,23, 24 or 25 nt. Region (1) and region (2) of the upstream probe, and region (2) and region (3) of the downstream probe are not essentially of identical length, the primary technical feature is the appropriate $\Delta T_m$ to each other, as described in table 1. The same applies for all probe combinations and their use in the method of the present invention, in particular for a probe combination of alternative a), b) and/or c) and the respective kit comprising the probes. Preferably for detection of AML biomarker(s).

In a third aspect of the method of the present invention, the increase or decrease of the hybridization rate, in particular of the downstream probe or upstream probe, is mediated by a competitive hybridization of the partially overlapping regions (2) of the at least one downstream and the at least one upstream probe to the respective complementary sequence, in particular on the at least one target with (T1-v) and/or without at least one analyte (T1-w). The same applies for all probe combinations and their use in the method of the present invention, in particular for a probe combination of alternative a), b) and/or c) and the respective kit. Preferably for detection of AML biomarker(s).

The preferred binding (hybridization) - equilibrium as defined above - of the downstream probe to T1-w is facilitated by means of a $\Delta$Tm [sequence region (3) binding on T1-w / to Tm sequence region (3) binding on T1-v] of equal to or greater than 4 °C, preferably 6, preferably higher than 6. The whole probe has a length of preferably at least 10 nucleotides (nt) up to 30 nt, preferably at least 12, 13, 14, 15, 16, 17, 18 up to 25, 26, 27, 28 nt. A range of at least 15 nt up to 30 nt is appropriate. Preferably the difference of length between the upstream probe and downstream probe is less than 10 nucleotides, less than 9, 8, 7 6 or less. It is possible and feasible to have a downstream probe and upstream probe of the same length. The aforementioned applies for alternatives a), b) and c) of the probes as described herein. Preferably the Tm of each probe is in the range of 55 to 75 °C, preferably in the range of 68-70 °C for use in a method comprising a PCR, preferably for the method performed in a CE device (see table 1). It is clear to the skilled person, that the length of each region and the resulting whole length is dependent on the primary technical feature of the appropriate $\Delta T_m$ to each other, as described in table 1.

The aforementioned decrease or increase of the hybridization rate of the respective probe to the respective complementary sequence of the at least one target sequence is visualized by the detection of the achieved signals. The achieved signals are used in the following to determine the relative quantities of T1-w and T1-v as described herein.

A target sequence (T1) can comprise more than one analyte and/or variations (T1-v) that are targeted with two differentially labelled competing probes according to the present invention. Differentially labelled means, that the hydrolysis product of both probes differs in color and/or migration behavior in capillary gel electrophoresis and can therefore be analyzed simultaneously. The target sequence is amplified by a forward and reverse primer (R, F) that frame (flank) the probes' binding site according to the present invention and facilitate equal amplification for wildtype (T1-w) and variations (T1-v) in the genetic hotspot site and achieves the amplicons A1-w and A1-v described herein. The competition consists of an overlap in the probes' annealing sequence (region (2)). In particular, the 3' portion of the inventive upstream probe overlaps with the 5' portion of the inventive downstream probe. This zone of competition spans over several nucleotides depending on the target sequence nucleotide composition. The downstream probe binding affinity changes depending on the presence of any genetic variation in the target sequence complementary to region (3) of the downstream probe (Fig. 1 A). This results in a preferential binding of the upstream probe and less efficient binding of the downstream probe. Therefore, the detected probe hydrolysis signal changes from preferentially downstream probe signal (F1-down) in wt targets (T1-w) to preferentially upstream probe signals (F1-up) in targets containing the respective variation (T1-v). Hence, probe affinity changes allow for detection of variations in the target sequence.

In a fourth aspect of the method of the present invention, the method further comprises a non-discriminatory amplification of the respective sequence, preferably with the at least one primer pair (F, R) and in particular by means of "real-time", in particular continuous real-time, PCR, or by means of an "end point PCR". Preferably, in a method using a probe combination a), b) and7or c). "Real-time PCR" comprises quantitative real-time PCR, quantitative PCR with CE (e.g. Modaplex) and "end point PCR" comprises end point detection, end point detection by means of a CE (e.g. Modaplex), end point detection in partitions based PCR approaches, digital PCR (comprising nanofluidic or DNA chip technologies) and digital droplet PCR (ddPCR). In another embodiment the present invention comprises separation of the obtained hydrolysis products (F1-Up, F1-Down) and optionally separation of the obtained amplicons A1-v and A1w, which are achieved by means of the non-discriminatory amplification. Preferably, the separation is performed by capillary electrophoresis (CE). More preferably for the method, being performed by means of a "real-time" PCR with a CE, alternative a) of the at least one downstream probe and/or of the at least one upstream probe is used.

In one embodiment at least one (F or R) or both primer are labeled. Preferably the label is a fluorophore. The at least one or both labeled primer are used in the embodiment of the method, wherein the amplified products A1-w and A1-v are detected. This enables to detect and distinguish between the type of variation.

The method according to any one of the described embodiments of the present invention, wherein each probe has a melting temperature (Tm) which is larger than the Tm of each primer of the at least one primer pair. Or wherein each primer of the primer pair has a melting temperature (Tm) which is lower than the Tm of the probes. Preferably, in the method of any one of the embodiments, the Tm of each probe is in the range of 55°C to 75 °C, preferably in the range of 68°C to 70 °C. Preferably, these ranges are used for a method comprising real-time PCR with a CE for separation. Most preferably it is a continuous real-time PCR performed within a CE device, such as the Modaplex device.

The method according to any one of the described embodiments of the present invention, wherein the $\Delta$ Tm primer to Tm probe is greater than or equal to 3 °C, preferably 4, more preferably 5. Preferably the melting temperature (Tm) of each primer of the primer pair is in the range of greater than or equal to 60 °C to less than or equal to 63 °C.

In a fifths aspect of the method of the present invention, the method preferably distinguishes three alternatives, wherein

- according to an alternative a) the at least one upstream probe and the at least one downstream probe comprises respectively (iii) a protective group (3'-blocker) at the 3'-end of probe inhibiting a primer extension reaction and (iv) at least one cleavable hydrolysis product from the 3'-end of the respective probe comprising at least one internal nuclease blocker at the 5'-region of the hybridizing sequence of the probe conferring resistance to a nuclease activity and structurally dividing a cleavable hydrolysis product from the 3'-end of the respective probe which comprises at least one nucleotide, and (v) at least one label bridged via a linker to the 5'-end of cleavable hydrolysis product of at least one probe or

- according to an alternative b) the upstream probe and the downstream probe comprises (iii) a protective group (3'-blocker) at the 3'-end of the probe inhibiting a primer extension reaction and which has a quencher function, preferably being capable of accepting photoluminescence resonance energy transfer, and (v) the at least one upstream probe and the at least one downstream probe comprises a label wherein the labels in relation to each other are different or

- according to an universal alternative c) the upstream probe and the downstream probe comprises (iii) a protective group (3'-blocker) at the 3'-end of the probe inhibiting a primer extension reaction and which has a quencher function, the at least one upstream probe and the at least one downstream probe comprise (iv) at least one cleavable hydrolysis product from the 3'-end of the probe comprising at least one internal nuclease blocker at the 5'-region of the hybridizing sequence of the downstream probe conferring resistance to a nuclease activity and structurally dividing a cleavable hydrolysis product from the 3'-end of the downstream probe which comprises at least one nucleotide, and (v) the at least one upstream probe and the at least one downstream probe comprises a label wherein the labels in relation to each other are different.

Each alternative a), b) or c) is compatible with each embodiment of the present invention, if not otherwise stated (e.g. specific of qPCR or specific for Modaplex). In general, alternative a) of the at least one upstream probe and the at least one downstream probe is tailored for a real-time PCR, in particular a quantitative and preferably continuous real-time PCR, and separation by capillary electrophoresis (CE). More preferably alternative a) is tailored for CE devices, such as the Modaplex system (Biotype GmbH) and other systems of Thermo Fisher Scientific Inc., Promega Corp, Fullerton, Beckman Coulter, Qiagen GmbH, Syntol, Agilent Technologies Inc. and others. For these alternatives at least one internal nuclease blocker is selected from a Backbone Modification as described herein, preferably from a LNA, BNA, ENA, BNA3, PMO or PNA, more preferably it is a LNA. In general alternative b) of the at least one upstream probe and the at least one downstream probe is tailored for a digital PCR, such as droplet digital PCR, plate-based digital PCR or chip-based digital PCR. Alternative c) is suitable for any method mentioned before. However, alternatives a), b) and b) of the probes enable all steps of the method according to the present invention and in particular the step of hybridization, if desired amplification of the targets, cleavage of the probes and detection of the achieved hydrolyses products.

In another embodiment the method of the present invention further comprise a step of providing at least one reference (synonym: reference nucleic acid sequence) representing at least one target sequence without a variation (T1-w) or the signals representing the hydrolysis products of T1-w. The reference may be a reference nucleic acid sequence material (synonym: reference material) - measured by performing the method - or a digital reference nucleic acid sequence information (synonym: digital reference sequence or digital reference) - which may be provided/uploaded onto the respective device, such as Modaplex. In a preferred embodiment, the reference is a digital reference T1-w or the respective signals representing the hydrolysis products of T1-w. In relation to said reference the released hydrolysis products (F1-Up, F1-Down) are detected and the presence of at least one variation (and/or analyte) is confirmed.

Therefore, in a sixth aspect of the present invention, the method, in particular for alternatives a), b) and c) described herein, further comprises a step of

calculation of the ratio between the upstream and downstream hydrolysis product(s) (F1-Up, F1-Down) on the target without variation (T1-w), the same ratio between the upstream and downstream hydrolysis product(s) (F1-Up, F1-Down) on the target with the at least one variation (T1-v) and comparison of the ratios (F1-Up, F1-Down [T1-w] and F1-Up, F1-Down [T1-v]) representing the degree of variation of the at least one molecular genetic analyte. In particular for the detection by means of NGS the degree of variation is referred to as variant allele frequencies (VAF).

For the calculation of the ratio as described above, different raw data are suitable. Provided a digital PCR, in particular droplet digital PCR and preferably with probe combination b) or c), is used, the "fractional abundance" as parameter is used for the calculation the ration of T1-v to T1-w. In contrast, provided a real-time PCR with a CE device, such as the Modaplex system and preferably with probe combination a) or c), is used, the area of the respective peak representing hydrolysis products F1-up and F1-down are used. An example is given based on the data achieved within the examples of the present invention. The calculation is described and explained in more details as follows:

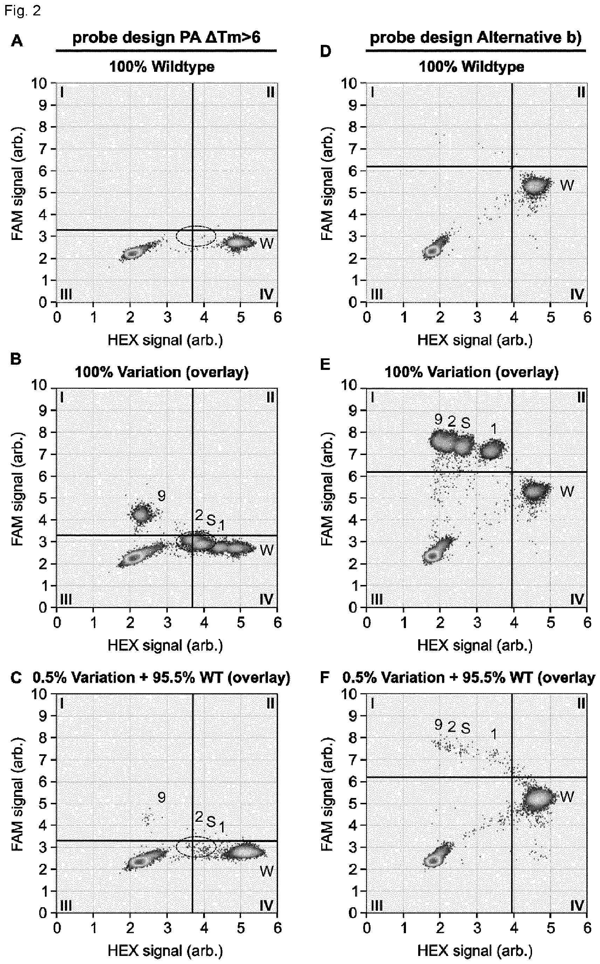

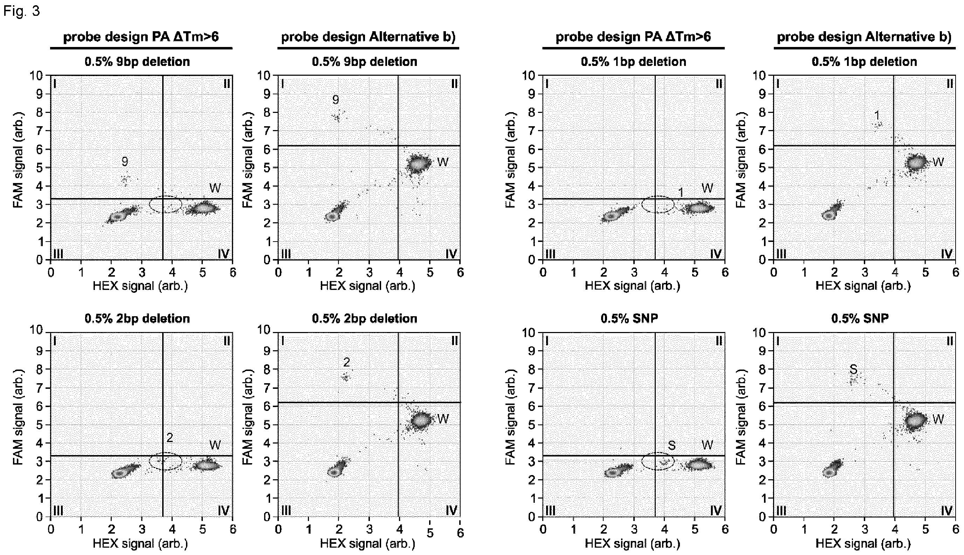

The fractional abundance (as shown in Fig. 2 and 3) can be based on concentrations of counts in a volume for partitioned PCR approaches.

$$Fractional\ Abundance = \left(\frac{F1_{up}}{F1_{up}\ +\ F1_{down}}\right) \times 100$$

Example for fractional abundance calculation based on the raw data of example summarized in table 3:

$$F1_{up}\ (T1\text{-}w) = 1\ RU/\mu l$$

$$F1_{down}\ (T1\text{-}w) = 301\ RU/\mu l$$

$$F1_{up}\ (T1\text{-}v;\ SNP,\ 50\%) = 204\ RU/\mu l$$

$$F1_{down}\ (T1\text{-}v;\ SNP,\ 50\%) = 147\ RU/\mu l$$

*RU = relative units*

$$Fractional\ Abundance\ (T1-w) = \left(\frac{1\ \frac{RU}{\mu l}}{1\frac{RU}{\mu l} + 301\ \frac{RU}{\mu l}}\right) \times 100\ \approx 0.33$$

$$Fractional\ Abundance\ (SNP, 50\%) = \left( \frac{204\ \frac{RU}{\mu l}}{204\frac{RU}{\mu l} + 147\ \frac{RU}{\mu l}} \right) \times 100\ \approx 58.12$$

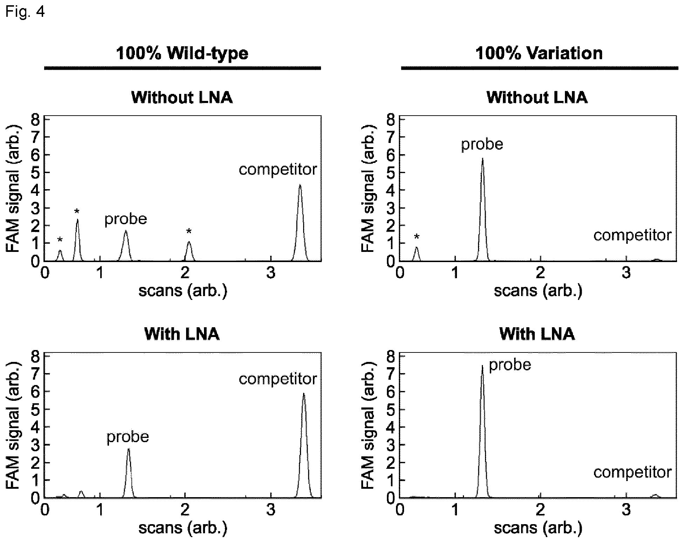

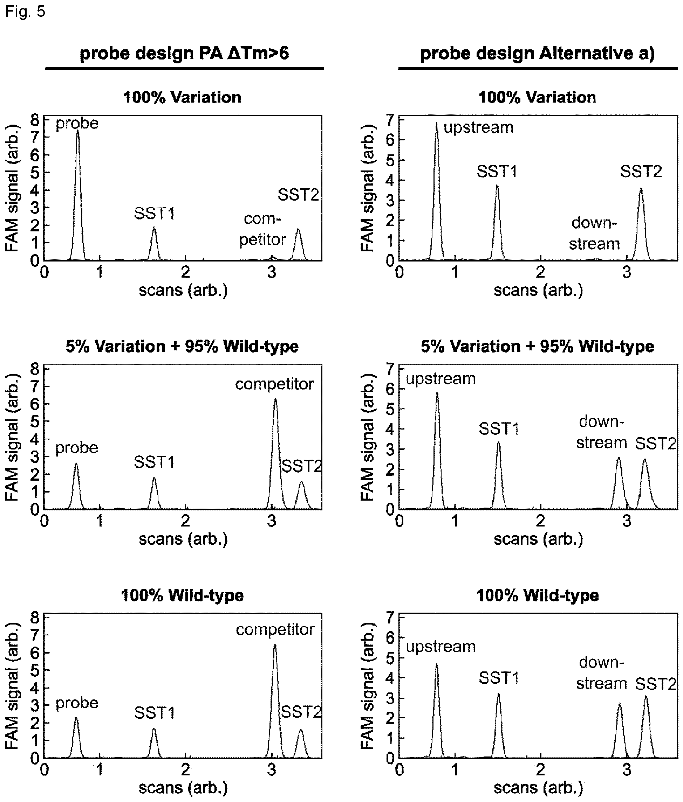

A ratio can be based on values of the area under the curve in electropherogram after PCR with concomitant capillary electrophoresis (as shown in Fig.4 and 5).

$$Ratio = \frac{F1_{up}}{F1_{down}}$$

Example for ratio calculation based on the raw data of example summarized in table 3:

$$F1_{up}\ (T1\text{-}w) = 353750\ RFU$$

$$F1_{down}\ (T1\text{-}w) = 247450\ RFU$$

$$F1_{up}\ (T1\text{-}v;\ SNP,\ 50\%) = 394600\ RFU$$

$$F1_{down}\ (T1\text{-}v;\ SNP,\ 50\%) = 102000\ RFU$$

*RFU = relative fluorescence units*

$$Ratio\ (T1 - w) = \frac{353750\ RFU}{247450\ RFU}\ \approx 1.43$$

$$Ratio\ (T1 - v;\ SNP, 50\%) = \frac{394600\ RFU}{102000\ RFU}\ \approx 3.87$$

As mentioned herein, the method according to the present invention can be tailored for at least two different alternatives a) and b) which predetermine the structure and amount of hydrolysis products and by means of which device said hydrolysis products can be detected as distinct and clear signals. More specifically, alternative a) enables hybridization of the downstream and upstream probe to the respective target sequences, cleavage by means of a nuclease to achieve one corresponding hydrolysis product for each hybridized probe and detection of said hydrolysis product, preferably in a CE, by a distinct and clear signal. Compared thereto, alternative a) enables hybridization of the downstream and upstream probe to the respective target sequences, cleavage by means of a nuclease which cleaves unspecific resulting in several hydrolysis products for each hybridized probe.

Therefore, in a seventh aspect of the method of the present invention, in alternative a), in particular a) and/or c), a non-discriminatory amplification of the respective sequence by means of a real-time PCR, in particular a quantitative and preferably continuous real-time PCR (qPCR), and a step of separation of the achieved hydrolysis products (F1-Up and/or F1-Down), preferably by capillary electrophoresis, are performed. The same is feasible with alternative c), if desired. Quantitative real-time PCR encompasses the PCR technologies as suitable for capillary electrophoreses (CE) devices, such as the Modaplex system (Biotype GmbH) and other systems of Thermo Fisher Scientific Inc., Promega Corp, Fullerton, Beckman Coulter, Qiagen GmbH, Syntol, Agilent Technologies Inc. and others. In a preferred embodiment of the method with a probe combination of alternative a), a non-discriminatory amplification of the respective sequences by means of a real-time, preferably quantitative real-time, PCR in combination with a step of separation of the achieved hydrolysis products (F1-Up and/or F1-Down) by CE are performed, most preferably in CE device such as Modaplex (Biotype GmbH, Dresden). Thus, for this embodiment of the method, the probe combination of alternative a) as explained herein is preferred (Fig. 1 D). However, the universal probe combination of alternative c) is also feasible for a real-time PCR in combination with a CE, in particular for the Modaplex device.

Compared thereto in aspect eight of the method of the present invention, in alternative b) a step of partitioning of the reaction mixture and a non-discriminatory amplification by means of an end point PCR are performed. The same is feasible with alternative c), if desired. The alternative of the method encompasses digital PCR, droplet digital PCR and end point PCR technologies as suitable for devices QX one (BioRad, USA), QIAcuity One (Qiagen, Germany), Applied Biosystems QuantStudio Absolute Q (ThermoFisher, USA) and others (Tan et al. 2022), as well as next generation sequencing technologies as suitable for e.g. NGS devices MiSeq (Illumina, USA) and NextSeq 550 (Illumina, USA). Thus, for this embodiment of the method, the probe combination of alternative b) and/or c) as explained herein is preferred (Fig. 1D) for digital PCR, droplet digital PCR and next generation sequencing technologies. However, the universal probe of alternative c) is also feasible for these technologies.

A "partition" within the meaning of the present invention is to be understood in context of a digital PCR and means a reaction mixture in defined cavities or reaction areas in a way that maximum one or two or no copies of the analyte are contained in each reaction unit. In embodiments of a digital PCR technology the method of the present invention further comprise a step of separation/partitioning to create and to provide partitions as defined herein. Therefore, in an embodiment of the present invention, the method comprises a step of creating at least one or more partitions that respectively comprise at least one target sequence T1-v with at least one variation - as defined herein - and at least one target sequence T1-w without a variation, wherein each partition comprise the probe combination of alternative b) or c) and, in particular all components necessary for the performance of the method of the present invention. According to the embodiment comprising partitions, at least the steps of hybridization, amplification and/or detection takes place in each partition. The step of calculation is performed with raw data for each partition. A plurality of partitions are possible according to the present invention with the probe combination of alternative b) or c). The skilled person knows how to perform a dPCR or ddPCR with a probe combination of alternative b) or c) of the present invention.

In aspect nine of the method of the present invention, at least one internal nuclease blocker, preferably one or more LNA, is located in region (2) of downstream probe and at least one internal nuclease blocker, preferably one or more LNA, in region (1) of the upstream probe. The probes for both alternatives a) and b) may have at least one internal nuclease blocker, respectively. In particular for alternative a) it is preferred that the at least one downstream probe and the at least one upstream probe comprise at least one internal nuclease blocker respectively, wherein it is located in region (2) of downstream probe and in region (1) of the upstream probe. However, it is important to note that downstream probes and/or upstream probes comprising at least one nuclease blocker are also suitable for the method according to the present invention and in particular also for alternative b) and/ or c) and that this embodiment is also part of the present invention.

In another aspect of the method of the present invention, in particular wherein a probe combination of alternative a), b) and/or c is provided, variation, within the at least one target sequence with the at least one variation (T1-v), comprises

- at least one deletion of at least one base pair,
- at least one insertion of at least one base pair,
- at least one deletion-insertion polymorphism (DIP), and/or
- at least one single nucleotide polymorphism (SNP), in particular comprising transversion and transition, in particular the SNP is a transversion or the SNP is transition.

In particular in comparison to the at least one target sequence without a variation (T1-w) or compared to a standard (or reference) which is provided within the method of the present invention said variations are detected and identified. The method, in particular for alternative a), b) and/or c), detects the listed variations above, preferably AML biomarker(s), and preferably

- a deletion of one, two, three, four, five, six, seven, eight, nine or more base pairs,
- an insertion of one, two, three, four, five, six, seven, eight, nine or more base pairs,
- a deletion-insertion polymorphism (DIP) of one, two, three, four, five, six, seven, eight, nine or more base pairs and/or
- a single nucleotide polymorphism (SNP) comprising transversion and transition.

The skilled person knows that a transversion encompasses point mutation within a class of bases, such as one purine base is replaced by another purine base or one pyrimidine base is replaced by another pyrimidine base. A transversion is when a purine base is replaced by a pyrimidine base or vice versa.

Any of the variation cause a mismatch for each of the probes a), b) or c) design as described herein. The mismatch may be partial and followed by hybridization of the 3'-end of the downstream probe or the mismatch may over the whole length of region (3) of the downstream probe so that only region (2) of the downstream probe still hybridizes. Such mismatches my result from a deletion, insertion and/or DIP corresponding to the length of region (3).

In a preferred embodiment of the method according to the present invention, more preferably for alternative a) and/or c), the least one variation is or comprise a SNP or a one bp (1bp) deletion. The SNP or 1 bp deletion is on the at least one target sequence with at least one variation (T1-v). Preferably, the method or the combination of probes according to the present invention, comprises at least one downstream probe comprising preferably at least one, two, three or four internal nuclease blocker.

In general, within the meaning of the invention, in particular for alternative a), b) and/or c), the at least one internal blocker, most preferably LNA, improves the specificity of the probe to the respective target sequence and influences the Tm of the respective probe. Thus, it is preferred to integrate at least one or more internal blockers, most preferably LNAs, in order to improve specificity of the respective probe. At the same time it can be used to increase Tm of the probe and to adjust the desired ∆ Tm between the regions ((1)/(3)), between the regions and targets ((3)/T1-w, (3)/T1-v), between the complete probes (upstream probe/downstream probe) and/or between the probes and primers in order to realize the method according to the invention.

At least one or more types of variations can be detected in the same reaction, in particular in the same run of the method of the present invention, simultaneously and be visualized as shown in Fig. 4 and 5. Provided the variation comprises at least 3 bp it is possible to identify a length polymorphism in a Modaplex as well. For that additionally, a labeled primer pair is used as described herein and the obtained amplified products, amplicon A1-w and amplicon A1-v, are detected. The comparison of the amplicons allows to identify a length polymorphism of the achieved amplicon of T1-v.

TaqMan™ probe-based detection in qPCR utilizes specific probes for either wildtype or mutated sequences. Thus, the TaqMan™ probes are specifically designed for both variants of the target sequence. In opposite, the probes according to the present invention are designed solely based on the wildtype sequence of the target site. A discrimination between wildtype and mutated allele status (variation) is achieved by probe binding affinity or by the hybridization rate as explained herein and therefore according to the present invention the target wildtype sequence is required, only. The binding affinity switches in case of sequence alteration on the target site. In result, probes of the present invention detect various mutations on the target site, while specific TaqMan™ probes are only sensitive for their specific target sequence to which they were designed.

In another aspect of the method of the present invention, in the method, in particular of alternative a), b) and/or c) respectively, a plurality of downstream probes and a respective plurality of upstream probes are used to detect a plurality of analytes, preferably for detection of AML biomarker(s). In particular a plurality of alternative a) or c) of downstream probes and a plurality of alternative a) upstream probes are used to detect a plurality of analytes, preferably for use in a CE device as described herein. In another embodiment a plurality of alternative b) or c) of downstream probes and a plurality of alternative b) upstream probes are used to detect a plurality of analytes, preferably for use in a digital PCR device as described herein. The methods allows a high multiplex degree by combining at least different modifications, spacer and labels, as described herein. "multiplex" refers to the detection of multiple different analytes, preferably within different target sequences, of at least 10, at least 20, at least 21, at least 22, at least 23, at least 24, at least 25, at least 30 or more targets, e.g., at least 50, at least 100, at least 250 or more targets.

In another embodiment a plurality of downstream probes of alternative a) or b) or of an alternative c) - as described herein - and a plurality of upstream probes of alternative a) or b) or of an alternative c) - as described herein - are used to detect a plurality of analytes. This mixed combination is suitable at least for a digital PCR device as described herein. The universal alternative c) is suitable without any further adjustment for use of real time and end-point PCR technologies for the method according to the present invention. Alternative c) of the probe combination according to the present invention is feasible for use in a digital PCR, digital droplet PCR, real time PCR, real time PCR in combination with a capillary electrophoreses, preferably the Modaplex device, and for next generation sequences (NGS).

In table 1 the rules or guidelines are described in more detail how a downstream probe and a upstream probe according to the present invention is designed. The rules give the skilled person sufficient guidance to design an upstream oligonucleotide probe and competitive downstream oligonucleotide probe of alternative a) or b) according to the present invention, for use in the method of the present invention, to provide a combination of probes according to the present invention and all other aspects and embodiments of the present inventions. The same rules apply accordingly for alternative c) of the probes, its combination and use in the method of the present invention.

Table 1: Design rules of probes according to the present invention.

| | Rule | Alternative a) for e.g. qPCR and CE Such as Modaplex | Alternative b) for e.g. digital PCR |
|---|---|---|---|
| 1 | A primer pair flanking the target region is chosen, wherein the known wildtype nucleic acid sequence (T1-w) is considered, only. | | - |
| 2 | Choose a suitable primer Tm, the Tm of the probe should be higher than the Tm of the primer. | Relevant technical feature is a ΔTm ≥ 5°C between probe and primers. In example 3 the primer sequence is adjusted accordingly to achieve a Tm between 60 °C and 63°C. | Relevant technical feature is a ΔTm ≥ 5°C between probe and primers. In example 1 the primer sequence is adjusted accordingly to achieve a Tm between 60 °C and 63°C. |
| 3 | Determine the region (3) of the downstream probe to cover the potential variation on the target sequence (T1-v). Select a sequence length of 5-20 nt. | Preferably, with a Tm of 30-55°C, more preferably 38-45°C for region (3) on the wt sequence. | Preferably, with a Tm of 30-55°C, more preferably 38-45°C for region (3) on the wt sequence. |
| 4 | Calculate the Tm within the region (3) of downstream probe only, when it binds the perfect matching complementary wild type sequence and additionally when it binds to T1-v with a mismatch to the variation with the lowest expected affinity change. | In this example, a SNP (resulting mismatch, e.g. Fig. 6) would change the Tm minimally compared to a perfect match on the wt sequence and is therefore preferred for Tm calculation on T1-v. The Tm difference can be calculated with tools such as the IDT OligoAnalyzer™ and is based on commonly accepted methods (SantaLucia and Hicks, Annu. Rev. Biophys. Biomol. Struct, 33, 415–440). If the exact variant is unknown choose a putative SNP for the Tm calculation on T1-v. | In this example, a SNP or 1nt Deletion (resulting mismatch, e.g. Fig. 6) would change the Tm minimally compared to a perfect match on the wt sequence and is therefore preferred for Tm calculation on T1-v. The Tm difference can be calculated with tools such as the IDT OligoAnalyzer™ and is based on commonly accepted methods (SantaLucia and Hicks, Annu. Rev. Biophys. Biomol. Struct, 33, 415–440). If the exact variant is unknown choose a putative SNP for the Tm calculation on T1-v. |
| 5 | Calculate ΔTm based on the Tm of region (3) of the downstream probe on T1-w and T1-v: if ΔTm <6°C, add at least one, two or three internal blocker[3] into the sequence of the downstream probe nucleotides at region 3.1 to increase the ΔTm. | Preferably an LNA or others are used as described herein[3]. In the herein described method, LNA's are used to increase the Tm. of the said probes and enhance sensitivity. Preferably is LNA placement on variant matching nucleotides (region 3.1). BUT as above, the probe does not comprise the complementary sequence to the variation (VAR, Fig. 1 C). Both probes comprise the complementary wt sequence. | Internal blockers are not necessary but the same design and embodiments are feasible. |
| 6 | Determine the overlapping region (2) of the downstream probe and upstream probe selecting a sequence of 5-20 nt and a Tm which is comparable to Tm of region (3) on the wt sequence (T1-w). | Preferably, with a Tm of 30-55°C, more preferably 38-45°C for region (2) on the wt sequence (T1-w). | Preferably, with a Tm of 30-55°C, more preferably 38-45°C for region (2) on the wt sequence (T1-w). Designs for b) do not require internal blocker, preferably LNA, on downstream probe 5' end. However, it is recommended to include LNA on downstream probe 5' end, located in region (2), to shift hybridization rate towards downstream probe on T1-w. |
| 7 | Starting from the overlapping sequence region (2) – which is also the 3' end of the upstream probe – determine the upstream region (1) by selecting 5-20 nt and a Tm which is similar or identical to Tm of region (2) and (3). | Preferably, with a Tm of 30-55°C, more preferably 38-45°C for region (1) on the wt sequence. Internal blocker, preferably LNA, on the upstream probe 5' end must be included in Tm calculation. | Preferably, with a Tm of 30-55°C, more preferably 38-45°C for region (1) on the wt sequence. |

| 8 | Finalize probe design for upstream and downstream probe with probe design rules: Choose label (Fluorophore), /Linker, add the at least one required internal blocker*3 to obtain the hydrolysis product, calculate the Tm of the complete probe and adjust the sequence length to yield a Tm which is larger than Tm of the primer, as described above of 68 – 70°C, add a 3′-blocker or any modification that stops the probe from acting as a primer at the 3′ end. | Preferably, Tm of the whole probe is in the range 55-75, more preferably 68-70 °C. Integrated nuclease blocker, preferably LNA, on the upstream probe 5′ end must be included in Tm calculation. Fluorophore for both probes can be identical or different and may determine the necessary amount of internal blockers. Preferably, with a Tm of 30-55°C, more preferably 38-45°C for region (2), (1) and (3) on the wt sequence. | Tm range is not limited, but rather depends on the desired device as described herein (dPCR, ddPCR, NGS or other) Depending on the device different labeling (Fluorophore) of each probe can be required to enable detection of separate signals (F1-up and F1-down). |
|---|---|---|---|
| 9 | Calculate and compare the final Tm of region (1), overlapping region (2) and region (3) assuming a perfect match on wt sequence (T1-w) and minimize the difference of Tm between regions (1), (2) and (3), preferably by sequence length adjustments and/or further modifications with blocker nucleotides. Finalize upstream and downstream probe design with design rules as listed in No. 9. The process in itself may need to be iterated. | Preferably, with a Tm of 30-55°C, more preferably 38-45°C for region (1), region (2) and region (3), respectively. Best performance was reached in the temperature range of 38°C-45°C for each individual part. Preferably, with a Tm in the range of 55-75°C, preferably 68-70°C for each probe. Internal blocker must be included in Tm calculation. To maintain the aforementioned rules, the process in itself may need to be iterated. | To maintain the minimal requirements of the aforementioned rules, the process in itself may need to be iterated, to obtain an optimal Δ of the Tm for region (2), (1) and (3) that is preferably <6°C. |

\* all regions (1), (2) and (3) are complementary to the respective sequence on the target sequence without a variation (wildtype, T1-w); *2 all Tm are always given based on a theoretical perfect and full hybridization (perfect match) with the complementary sequence of T1-w; *3 the internal blocker is selected from a Backbone Modification as described herein, preferably from a LNA, BNA, ENA, BNA3, PMO or PNA, more preferably it is a LNA.

The skilled person knows the design basics for "Blocker Displacement Amplification". This mainly includes the configuration of primer position and primer length based on the free enthalpy of the oligos and their compartments. Our experiments (data not shown) showed no correlation for probe designs. Designs using free enthalpy (G) calculations for either upstream or downstream competitor did not correlate with efficient target discrimination. In contrast, tailored designs based on the melting temperature of both competitors resulted in high discrimination rate for cycle threshold values (known as Ct or Cq) and relative fluorescence units (RFU) between signals on wildtype and mutated target variants. The probes of the present invention, in particular for real-time PCR and CE, require additional DNA modifications as described herein, such as LNAs. LNAs enhance the Tm of probes influencing the binding affinity of the inventive probes significantly. Tm-based designs of the inventive probes include this circumstance rendering them more suitable and reliable for the probes of the present invention. Furthermore, the probe design of the downstream region (3) additionally includes the Tm decrease that results from mismatches with the target sequence. The prior art technologies use the G variations for mismatches to choose the ΔTm (5'-3' or 3'-5') for design purposes only. Additionally, the individual probe signal intensities and hence the resulting relative signal ratios were shown to correlate with the probe concentrations in the PCR reaction. This design optimization step is not disclosed in the prior art. Thus presently, the present invention offers an opportunity to increase assay sensitivity, in particular for real-time PCR and CE, preferably for Modaplex.

Another aspect of the present invention is a combination of at least two oligonucleotide probes ("probe") comprising

- at least one upstream oligonucleotide probe ("upstream probe") comprising

     (i) a sequence region (1) and a sequence region (2), which together compose a sequence which is complementary to a sequence that is the same on the target sequence without (T1-w) and on the target sequence with at least one variation (T1-v), preferably region (1) and region (2) respectively have a sequence length of at least 5 nt up to 20 nt, more preferably and length of at least 5 nt up to 15 nt,

- at least one competitive downstream oligonucleotide probe ("downstream probe") comprising

     (i) a sequence region (3) and a sequence region (2), which together compose a sequence which is complementary to a sequence on the target sequence without a variation (T1-w), preferably region (3) and region (2) respectively have a sequence length of at least 5 nt up to 20 nt, length of at least 5 nt up to 15 nt and
     (ii) at least one mismatch within the region (3) in relation to the at least one variation on the target sequence (T1-v),

- the 3'-end of the upstream and 5'-end of the downstream probe are complementary to the partially overlapping region (2) on a sequence 3' upstream of the at least one variation on the at least one target sequence (T1-v), and

- the sequence region (1) and the sequence region (3) have a similar melting temperature (Tm) and a $\Delta$ Tm sequence region (1) to Tm sequence region (3) of less than or equal to 6 °C, wherein

 - according to an alternative a) the at least one upstream probe and the at least one downstream probe comprises respectively (iii) a protective group (3'-blocker) at the 3'-end of probe inhibiting a primer extension reaction and (iv) at least one cleavable hydrolysis product from the 3'-end of the respective probe comprising at least one internal nuclease blocker at the 5'-region of the hybridizing sequence of the probe conferring resistance to a nuclease activity and structurally dividing a cleavable hydrolysis product from the 3'-end of the respective probe which comprises at least one nucleotide, and (v) at least one label bridged via a linker to the 5'-end of cleavable hydrolysis product of at least one probe or
 - according to an alternative b) the upstream probe and the downstream probe comprises (iii) a protective group (3'-blocker) at the 3'-end of the probe inhibiting a primer extension reaction and which has a quencher function, in particular one single modification realizes both functions, preferably being capable of accepting photoluminescence resonance energy transfer, (iv) at least one cleavable hydrolysis product from the 3'-end of the respective probe (F1-Down and/or F1-up) comprising at least one nucleotide of the 5'-end of the respective probe and (v) the at least one upstream probe and the at least one downstream probe comprises a label wherein the labels in relation to each other are different or
 - according to a universal alternative c) the upstream probe and the downstream probe comprises (iii) a protective group (3'-blocker) at the 3'-end of the probe inhibiting a primer extension reaction and which has a quencher function, in particular one single modification realizes both functions, the at least one upstream probe and the at least one downstream probe comprise (iv) at least one cleavable hydrolysis product from the 3'-end of the probe comprising at least one internal nuclease blocker at the 5'-region of the hybridizing sequence of the downstream probe conferring resistance to a nuclease activity and structurally dividing a cleavable hydrolysis product from the 3'-end of the downstream probe which comprises at least one nucleotide, and (v) the at least one upstream probe and the at least one downstream probe comprises a label wherein the labels in relation to each other are different.

In a preferred embodiment of the probe combination a), b) and/or c), the whole respective probe has a length of preferably at least 10 nucleotides up to 30 nucleotides (nt), preferably at least 12, 13, 14, 15, 16, 17, 18 up to 25, 28 nt. A range of at least 15 nt up to 30 nt is appropriate. Preferably the difference of length between the upstream probe and downstream probe is less than 10 nucleotides, less than 9, 8, 7 6 or less. It is possible and feasible to have a downstream probe and upstream probe of the same length.

Preferably, the probe combination a), b) and/or c) is designed, that a $\Delta$ Tm [sequence region (3) binding on T1-w / to Tm sequence region (3) binding on T1-v] of equal to or greater than 4 °C, preferably 6, preferably higher than 6 is achieved, in order to facilitate a preferred binding (hybridization) - equilibrium as defined above - of the downstream probe to T1-w. Preferably the Tm of each probe is in the range of 55 to 75 °C, preferably in the range of 68-70 °C for use in a method comprising a PCR, preferably for the method performed in a CE device (table 1).

For the herein described method, design rules and performed examples, the Tm was calculated using the software IDT tool OligoAnalyzer™. The tool is available on the IDT homepage (www.idtdna.com). The instructions for use of the tool are available on the tool website (www.idtdna.com/calc/analyzer). Other suitable tools are e.g. Tm Calculator [ThermoFisher, USA] and Geneious Prime [Geneious, New Zealand]. To calculate the Tm, the oligosequence of the primer or probe must be provided in the field 'Sequence'. Modifications of the oligo must be marked according to the instructions and target type DNA must be selected. The parameters of the desired buffer and conditions must be provided in the desired calculation tool (as described herein). For the example described herein the "Modaplex Buffer 9" (Art. No. 85-20201, Biotype GmbH, Dresden, Germany) was considered for calculation and used in the method (Example 1 to 4) Mismatch Tm for the hybridized downstream probe are analyzed using the TM MISMATCH option in the tool. For the example calculations a putative SNP Mismatch (T1-v) on the complementary 3' to 5'-strand of the probe was inserted according to the instructions (Table 1).

In another embodiment the downstream and upstream probe are used in relative quantities or within a ratio of 0.1 to 10 with respect to each other, preferably in equimolar quantity. According to the invention it is not necessary to provide the downstream probe or the upstream probe in a significantly higher content to each other. Preferably, the method of the present invention comprises further a step of providing the at least one downstream probe and the at least one upstream probe, in particular in a combination, within the aforementioned quantities. The probe combination at a predetermined quantity may be provided within a predetermined reaction mixture or separately and can be added as a combination or as separate probes, individually, to the at least one target at the at least one primer pair at any desired time. Thus, in another embodiment of the method of the present invention, the method may comprise a step of providing at least one downstream probe and the at least one upstream probe, separately, preferably, adjusting their relative quantities or within a ratio of 0.1 to 10 with respect to each other and mixing with the at least one primer pair, the at least one target T1-w and potentially T1-v, and preferably with other agents necessary for the method are well known to the skilled person. In particular it is not necessary to provide the downstream probe in a significantly higher content to compared to the content of the upstream probe in order to realize detection of the variation. The aforementioned applies for all alternatives.

In one embodiment the combination of at least two oligonucleotide probes ("probe") is tailored for a real-time PCR, in particular a quantitative and preferably continuous real-time PCR, and separation by capillary electrophoresis. More preferably for real-time PCR alternative a) or c) of the probes as described here are used which are feasible for capillary electrophoreses (CE) devices, such as the Modaplex system (Biotype GmbH) and other systems of Thermo Fisher Scientific Inc., Promega Corp, Fullerton, Beckman Coulter, Qiagen GmbH, Syntol, Agilent Technologies Inc. and other. The combination of probes a) comprises

- at least one upstream oligonucleotide probe ("upstream probe") comprising

  (i) a sequence region (1) and a sequence region (2), which together compose a sequence which is complementary to a sequence that is the same on the target sequence without (T1-w) and on the target sequence with at least one variation (T1-v), preferably region (1) and region (2) respectively have a sequence length of at least 5 nt up to 20 nt, and
  (iii) a protective group (3'-blocker) at the 3'-end of probe inhibiting a primer extension reaction,
  (iv) at least one cleavable hydrolysis product from the 3'-end of the respective probe comprising at least one internal nuclease blocker at the 5'-region of the hybridizing sequence of the probe, preferably in region (1), conferring resistance to a nuclease activity and structurally dividing a cleavable hydrolysis product from the 3'-end of the respective probe which comprises at least one nucleotide,

- at least one competitive downstream oligonucleotide probe ("downstream probe") comprising

  (i) a sequence region (3) and a sequence region (2), which together compose a sequence which is complementary to a sequence on the target sequence without a variation (T1-w), preferably region (3) and region (2) respectively have a sequence length of at least 5 nt up to 20 nt,
  (ii) at least one mismatch within the region (3) in relation to the at least one variation on the target sequence (T1-v),
  (iii) a protective group (3'-blocker) at the 3'-end of probe inhibiting a primer extension reaction,
  (iv) at least one cleavable hydrolysis product from the 3'-end of the respective probe comprising at least one internal nuclease blocker at the 5'-region of the hybridizing sequence of the probe, preferably in region (2), conferring resistance to a nuclease activity and structurally dividing a cleavable hydrolysis product from the 3'-end of the respective probe which comprises at least one nucleotide, and

- at least one probe or both comprise
  (v) at least one label bridged via a linker to the 5'-end of the cleavable hydrolysis product,
- the 3'-end of the upstream and 5'-end of the downstream probe are complementary to the partially overlapping region (2) on a sequence 3' upstream of the at least one variation on the at least one target sequence (T1-v), and
- the sequence region (1) and the sequence region (3) have a similar melting temperature (Tm) and a $\Delta$ Tm sequence region (1) to Tm sequence region (3) of less than or equal to 6°C.

The same is adaptable for alternative c). These probe combinations are used in the method according to the present invention to obtain the respective released, optionally labeled, hydrolysis products which respectively are the confirmation of the presence of at least one analyte. If both probes are labeled, the label may be identical or different. Preferably, the above combination of probes is combined with at least one primer pair (F, R) which preferably is labeled, preferably with a fluorophore. The labeled primer pair, in particular with the probe combination, are for use to obtain at least one labeled amplicon of A1-v (based on amplification of T1-v) and at least one labeled amplicon A1-w (based on amplification of T1-v). The comparison of A1-v and A1-w, in particular the size as defined by their length of nucleic acid sequence, enables the confirmation of a specific variation of the at least one analyte. In particular of a deletion or insertion of at least 3 bp. Thus, based on said released, optionally labeled, hydrolysis products according to alternative a) in combination with the amplicons a qualitative and relatively quantitative analysis of a variation within the analyte is possible.

In another embodiment the combination of at least two oligonucleotide probes ("probe") is tailored for a digital PCR, such as droplet digital PCR and/or NGS. More preferably for digital PCR alternative b) of the probe as described herein are used which are feasible for devices for digital PCR and NGS, as described herein. The combination of probes b) comprises

- at least one upstream oligonucleotide probe ("upstream probe") comprising

  (i) a sequence region (1) and a sequence region (2), which together compose a sequence which is complementary to a sequence that is the same on the target sequence without (T1-w) and on the target sequence with at least one variation (T1-v), preferably region (1) and region (2) respectively have a sequence length of at least 5 nt up to 20 nt, and
  (iii) a protective group (3'-blocker) at the 3'-end of probe inhibiting a primer extension reaction and which has a quencher function, preferably being capable of accepting photoluminescence resonance energy transfer,
  (iv) at least one cleavable hydrolysis product from the 3'-end of the respective probe (F1-Down and/or F1-up) comprising at least one nucleotide of the 5'-end of the respective probe and
  (v) a label at the 5'-end of the cleavable hydrolysis product

- at least one competitive downstream oligonucleotide probe ("downstream probe") comprising

  (i) a sequence region (3) and a sequence region (2), which together compose a sequence which is complementary to a sequence on the target sequence without a variation (T1-w), preferably region (3) and region (2) respectively have a sequence length of at least 5 nt up to 20 nt,
  (ii) at least one mismatch within the region (3) in relation to the at least one variation on the target sequence (T1-v),
  (iii) a protective group (3'-blocker) at the 3'-end of probe inhibiting a primer extension reaction and which has a quencher function, preferably being capable of accepting photoluminescence resonance energy transfer,
  (iv) at least one cleavable hydrolysis product from the 3'-end of the respective probe (F1-Down and/or F1-up) comprising at least one nucleotide of the 5'-end of the respective probe and
  (v) a label at the 5'-end of the cleavable hydrolysis product

- wherein the label of the downstream and of the upstream probe are different,
- the 3'-end of the upstream and 5'-end of the downstream probe are complementary to the partially overlapping region (2) on a sequence 3' upstream of the at least one variation on the at least one target sequence (T1-v), and
- the sequence region (1) and the sequence region (3) have a similar melting temperature (Tm) and a $\Delta$ Tm sequence region (1) to Tm sequence region (3) of less than or equal to 6°C.

The same is adaptable for alternative c). These probe combinations are used is used in the method according to the present invention to obtained the respective released labeled hydrolysis products which respectively are the confirmation of the presence of at least one analyte. Based on said released labeled hydrolysis products according to alternative b) a qualitative and relatively quantitative analysis of a variation within the analyte is possible. Preferably, the above combination of probes is combined with at least one primer pair (F, R) which is labeled, preferably with a fluorophore. The labeled primer pair, in particular with the probe combination, are for use to obtain at least one labeled amplicon of A1-v (based on amplification of T1-v) and at least one labeled amplicon A1-w (based on amplification of T1-v).

Thus, another aspect of the present invention is at least one hydrolysis product released from the respective upstream probe (F1-Up) and at least one hydrolysis product released from downstream probe (F1-Down), preferably from probes of combination a), b) or c), according to the method as described herein. Preferably, the at least one hydrolysis product released from the respective upstream probe (F1-Up) and/or at least one hydrolysis product released from downstream probe (F1-Down), are labeled, more preferably with a fluorophore.

Another subject matter is the combination of the at least one released hydrolysis product from the respective upstream probe (F1-Up), preferably labeled, and the at least one hydrolysis product released from downstream probe (F1-Down), preferably labeled, together with at least one labeled amplicon A1-v, preferably labeled, and at least one labeled amplicon A1-w, preferably labeled. Respectively, for each probe design a), b) or c). The combination of F1-Up, F1-Downs, A1-w and A1-v, obtained by the method as described herein and for use for the detection and identification of at least one analyte.

A further aspect of the present invention is the use of the combination of at least one downstream probe and of the least one upstream probe - as described above for the combination of probes a), b) or c) and embodiments - and/or of the respective hydrolysis product - as described above and which are preferably labeled - in a method for the detection of at least one or more analytes according to the invention as described herein.

Another aspect of the use, the released hydrolysis product, preferably of the labeled released hydrolysis product from probes of alternative a), b) or c), respectively is the confirmation of the presence of at least one analyte.

Another aspect of the present invention is at least one labeled amplicon A1-v and at least one labeled amplicon A1-w, which in comparison are the confirmation of a specific variation of the at least one analyte for alternative a) as described herein. The same applies for c).

Another subject-matter is a kit, in particular for the use in the method of the first aspect of the present invention and of any of the described embodiments, comprising

- the combination of probes as described above, preferably a), b) or c),
- at least one primer pair (R, F) specific for the respective target to be amplified as described herein and
- optionally a reference representing T1-w enabling detection of the at least one molecular genetic analyte comprising a target sequence with at least one variation (T1-v)

The reference are information about a suitable digital reference available from a database (e.g., UCSC Genome Browser, NCBI Blast, Ensembl genome browser), a digital reference as defined herein provided within the kit, representing T1-w or its hydrolysis product, respectively for uploading to the respective device, preferably CE device, such as Modaplex or the reference is a reference material to be applied in the method of the present invention.

Any embodiment of each probe or combination of probes, of the primer pair and method - each alone or in any combination - apply accordingly for the kit.

The present invention provides in a preferred embodiment, preferably a fully automated and/or multiplex, method for the simultaneous detection of a plurality of molecular genetic analytes. The same method is suitable for simplex and multiplex analysis. The reaction mixture comprises at least one, preferably two, oligonucleotide probes with a cleavable hydrolysis product for use in said method. Through the method the plurality of cleavable hydrolysis products is specifically released by a nuclease from the respective probe. Following a separation step, preferably in a capillary electrophoresis, for each hydrolysis product a clear and distinguishable from others signal is achieved. Each separated hydrolysis product respectively results in a signal enabling the qualitative and/or relatively quantitative detection of each analyte comprising a molecular variant, e.g. single nucleotide polymorphism (SNP), deletion-insertion polymorphism (DIP) or other, respectively, that was targeted specifically by the plurality of probe combinations. Preferably, the method is suitable to detect qualitatively and/or quantitatively small molecular variants as such SNP.

Alternative a) of the at least one upstream probe and the at least one downstream probe is tailored for a real-time PCR, in particular a quantitative and preferably continuous real-time PCR, and separation by capillary electrophoresis as described herein. However, c) is also feasible. The method for this alternative a) for the detection of at least one or more molecular genetic analytes comprises

- providing a collective and continuous reaction setup reaction mixture comprising

  • at least one molecular genetic analyte comprising a target sequence (T1) without (T1-w) and potentially at least one molecular genetic analyte comprising a target sequence with at least one variation (T1-v),
  • a primer pair (F, R) flanking the at least one T1-w and the at least one T1-v being suitable for non-discriminatory amplification of any target sequence and preferably labeled,
  • at least one upstream oligonucleotide probe ("upstream probe") comprising

    (i) a sequence region (1) and a sequence region (2), which together compose a sequence which is complementary to a sequence that is the same on the target sequence without (T1-w) and on the target sequence with at least one variation (T1-v), Preferably said sequence is located 3'upstream of the sequence comprising the at least one variation (variant region) and preferably region (1) and region (2) respectively have a sequence length of at least 5 nt up to 20 nt, and

  • at least one competitive downstream oligonucleotide probe ("downstream probe") comprising

    (i) a sequence region (3) and a sequence region (2), which together compose a sequence which is complementary to a sequence on the target sequence without a variation (T1-w), preferably region (3) and region (2) respectively have a sequence length of at least 5 nt up to 20 nt, and
    (ii) at least one mismatch within the region (3) in relation to the at least one variation on the target sequence (T1-v), preferably the mismatch is flanked by the remaining sequence of region (3), preferably by at least one, more preferably two nucleotides

  • the 3'-end of the upstream and 5'-end of the downstream probe are complementary to the partially overlapping region (2) on a sequence 3' upstream of the at least one variation on the at least one target sequence (T1-v), and
  • the sequence region (1) and the sequence region (3) have a similar melting temperature (Tm) and a ∆ Tm sequence region (1) to Tm sequence region (3) of less than or equal to 6°C,

wherein **each** probe further comprises:

(iii) a protective group (3'-blocker) at the 3'-end of probe inhibiting a primer extension reaction,

(iv) at least one internal nuclease blocker at the 5'-region of the hybridizing sequence of the probe conferring resistance to a nuclease activity and structurally dividing a cleavable hydrolysis product from the 3'-end of the respective probe and wherein the cleavable hydrolysis product (F1-Down, F1-up) comprises

- at least one nucleotide of the 5'-end of the probe, and
- optionally a label bridged via a linker to the cleavable hydrolysis product of at least one probe, preferably at least one probe is labeled, alternatively both probes are labeled,

the method comprising the steps

- contacting the target sequences with the at least one downstream and the least one upstream probe and the at least one primer pair (F, R),
- hybridization of the at least one primer pair (F,R) and of at least one probe to its respective complementary sequence on a same strand of the same target sequence, wherein the respective probe hybridizes within the region flanked by the primer pair (F,R), and

- the upstream probe and downstream probe hybridize in equilibrium to the at least one target sequence without any analyte (T1-w),
- the downstream probe hybridizes with a decreasing hybridization rate in relation to the upstream probe to the target sequence with the at least one analyte (T1-v), and
- the upstream probe hybridizes with an increased hybridization rate, in particular mediated by the partial overlap of region (2) of both probes, in relation to the downstream probe to the target sequence with the at least one analyte (T1-v),

- cleavage of the hybridized probe(s) with a target-dependent 5' to 3' nuclease activity to release the at least one cleavable hydrolysis product (F1-Up and/or F1-Down),
- separation of the achieved hydrolysis products (F1-Up and/or F1-Down), preferably separation by means of capillary electrophoresis, and
- detection of the achieved hydrolysis product(s) (F1-Up and/or F1-Down), preferably relatively quantitative and qualitative detection and
- preferably calculation of the ratio between the upstream and downstream hydrolysis product(s) (F1-Up, F1-Down) on the target without variation (T1-w), the same ratio between the upstream and downstream hydrolysis product(s) (F1-Up, F1-Down) on the target with the at least one variation (T1-v), and more preferably comparison of the ratios (F1-Up, F1-Down [T1-w] and F1-Up, F1-Down [T1-v]) and achieving a difference, which represents the degree of variation of the at least one molecular genetic analyte.

Accordingly, the same method with the probe design c) can be performed. Preferably, the achieved hydrolysis product(s) (F1-Up and/or F1-Down) are representing the at least one molecular genetic analyte. The detection of the obtained hydrolysis products (synonym: probe signals), preferably in the Modaplex system, facilitate real time analysis, discrimination and quantitation of both signals (F1-Up and/or F1-Down) and more preferably an automated analysis of the two relative signals to determine the presence of variation.

Thus, in another embodiment of the method according to the present invention, a step of automated analysis is performed. Automated analysis includes automated calculation as explained herein, automated presentation of the calculated ratio and preferably automated presentation of the final result (diagnosis) which is a statement about the degree of variation and preferably type of variation

In another aspect of the method of present invention, the sequence region (1) of the upstream probe and the sequence region (3) of the downstream probe have a similar melting temperature (Tm) and $\Delta$ Tm sequence region (1) to Tm sequence region (3), based on a perfect match with T1-w, of less than or equal to 5°C, preferably less than or equal to 4°C, more preferably less than or equal to 3°C.

In another aspect of the method of present invention, with increasing $\Delta$ Tm of Tm sequence region (3) to Tm of the respective sequence on T1-v comprising the at least one analyte, the hybridization rate of the downstream to T1-v decreases. The preferred binding (hybridization) - equilibrium as defined above - of the downstream probe to T1-w is facilitated by means of a $\Delta$ Tm [sequence region (3) binding on T1-w to Tm sequence region (3) binding on T1-v] of equal to or greater than 4 °C, preferably 6, preferably higher than 6.

In another aspect of the method of present invention, region (3) of the at least one downstream probe is designed to result the at least one mismatch at the 5'-end of region (3) in relation to the at least one analyte on T1-v.

Preferably, in the method the at least cleavable hydrolysis product comprise a label bridged via a linker to the cleavable hydrolysis product, preferably at least one probe is labeled, alternatively both probes are labeled, wherein the label is a fluorophore. In another embodiment, preferably a competitive downstream prone is not labeled and the upstream probe is labeled. In another embodiment both probes, the at least one downstream and the at least one upstream probe, comprise a label, preferably a fluorophore, wherein the label may be different or identical for both, the upstream and downstream probe. Both hydrolysis products released from the probes comprising the same label can be detected and differentiated from each other in the same channel.

In another aspect of the method of the present invention, variation within the at least one target sequence with the at least one variation (T1-v) comprises

- at least one deletion of at least one base pair,
- at least one insertion of at least one base pair,
- at least one deletion-insertion polymorphism (DIP), and/or
- at least one single nucleotide polymorphism (SNP), in particular comprising transversion and transition.

In particular in comparison to the at least one target sequence without a variation (T1-w) or compared to a standard which is provided within the method of the present invention. In a preferred embodiment of the method according to the present invention, the least one molecular genetic analyte is or comprise a SNP or a one bp deletion. The SNP or one bp deletion is on the at least one target sequence with at least one molecular genetic analyte (T1-v). Preferably, the method or the combination of probes according to the present invention, comprises at least one downstream probe comprises preferably at least one, two, three or four internal nuclease blockers.

In another embodiment of the method the present invention, a plurality of downstream probes and a plurality of upstream probes are provided, and/or are used, to detect a plurality of analytes, wherein all respective cleavable hydrolysis products (F1-Down, F1-up) may comprise the same label coupled to the at least one nucleotide of each cleavable hydrolysis product (F1-Down, F1-up) and each comprise a different linker and/or at least one different modification.

Another subject-matter of the present invention is a combination of at least two oligonucleotide probes ("probe") of alternative a) comprising

- at least one molecular genetic analyte comprising a target sequence (T1) without (T1-w) and potentially at least one molecular genetic analyte comprising a target sequence with at least one variation (T1-v),
- a primer pair (F, R) flanking the at least one T1-w and the at least one T1-v being suitable for non-discriminatory amplification of the target sequences,
- at least one upstream oligonucleotide probe ("upstream probe") comprising

   (i) a sequence region (1) and a sequence region (2), which together compose a sequence which is complementary to a sequence that is the same on the target sequence without (T1-w) and on the target sequence with at least one variation (T1-v), and

- at least one competitive downstream oligonucleotide probe ("downstream probe") comprising

   (i) a sequence region (3) and a sequence region (2), which together compose a sequence which is complementary to a sequence on the target sequence without a variation (T1-w), and
   (ii) at least one mismatch within the region (3) in relation to the at least one variation on the target sequence (T1-v),

- the 3'-end of the upstream and 5'-end of the downstream probe are complementary to the partially overlapping region (2) on a sequence 3' upstream of the at least one variation on the at least one target sequence (T1-v), and
- the sequence region (1) and the sequence region (3) have a similar melting temperature (Tm) and a $\Delta$ Tm sequence region (1) to Tm sequence region (3) of less than or equal to 6°C,

wherein **each** probe further comprises:

   (iii) a protective group (3'-blocker) at the 3'-end of probe inhibiting a primer extension reaction,
   (iv) at least one internal nuclease blocker at the 5'-region of the hybridizing sequence of the probe conferring resistance to a nuclease activity and structurally dividing a cleavable hydrolysis product from the 3'-end of the respective probe and wherein the respective cleavable hydrolysis product (F1-Down, F1-up) comprises

- at least one nucleotide of the 5'-end of the probe, and
- a label bridged via a linker to the cleavable hydrolysis product.

The method or the combination of probes of alternative a) according to the present invention, wherein the respective probe comprises at least one modification of at least one nucleotide comprising backbone modifications, none-backbone modifications and/or artificial bases wherein

- backbone-modification comprises artificial modification at the 2'and/or 4' position of the five-carbon sugar of a nucleotide and
- non-backbone-modification comprises artificial chemical modification which is coupled to the 5'-end and/or to the 3'-end of the nucleotide.

The method or combination of probes of alternative a) according to the present invention, wherein the non-backbone-modification comprises spacers which are chemical structures coupled to the 3'- and/or 5'-end of a nucleotide or between two nucleotides and preferably selected from

a) alkyl alcohol of $(C_n)$-OH, wherein n is an integer and at least 3, preferably, 3, 6, 9 or 12 comprising propanyl (Spacer C3), hexanyl (Spacer C6), nonanyl (Spacer C9) and dodecanyl (Spacer C12).
b) glycol ether of $(C-O-C-C)_n$-OH wherein n is an integer and at least 1, preferably comprising triethylene glycol (Spacer 9), tetraethylene glycol (Spacer 12), and hexaethylene glycol (Spacer 18) and/or
c) a tetrahydrofuaran derivative containing a methylene group occupied in the 1 position of 2'-deoxyribose.

In another aspect of the method or of the combination of probes of alternative a), the downstream probe comprises at least one internal nuclease blocker and is selected from a Backbone Modification as described herein, preferably from a LNA, BNA, ENA, BNA3, PMO or PNA, more preferably it is a LNA. Preferably in the method of the present invention, the at least internal nuclease blocker is located in region (2) of downstream probe and in region (1) of the upstream probe. More preferably, the downstream probe comprises at least one internal nuclease blocker selected from an LNA, BNA, ENA, BNA3, PMO or PNA, preferably an LNA, located within a region (3.1) at the 5'-end of region (3) resulting at least one mismatch in region (3.1) in relation to the at least one variation of T1-v.

Another aspect of the present invention is a hydrolysis product (F1-Up) and a hydrolysis product (F1-Down) released from the respective upstream probe and downstream probe of alternative a) according to any one of the embodiments described herein. Another aspect is a use of at least one downstream probe and of the least one upstream probe as described herein and/or of a hydrolysis product as described herein in a method for the detection of at least one or more analytes according to any one of the embodiments of the present invention. In another embodiment of the use, the released hydrolysis products are the confirmation of the presence of at least one analyte in relation to a reference (synonym: reference nucleic acid sequence) which represent the sequence without a variation (T1-w) or its respective hydrolysis product. In a preferred embodiment, the reference is a digital reference as defined herein.

Contrary to the teaching of the prior art, wherein a Δ TM of > 6 between a first and a second competitive probe is required e.g. for digital PCR, presently it has been shown that in particular alternative a) of the inventive probe design, wherein the probes have a Δ TM of < 6, show feasible detection of T1-w and T1-v (Example 3, Fig. 5). "bulk" It is even feasible in a bulk PCR - in contrast to a partition as used for dPCR with a single target molecule - with a large amount of target sequences.

The method may further comprisess a non-discriminatory amplification of the respective sequence, preferably by means of a continuous PCR or end point PCR, more preferably performed by means of a CE device as described herein. In another embodiment the method obtain at least one amplicon A1-w and at least one amplicon A1-v.

Another aspect is the a least one achieved amplicon A1-v of T1-v and/or the a least one achieved amplicon of A1-w of T1-w, in particular obtained by non-discriminatory amplification with the at least one primer pair (F, R), wherein the detection (presence) of A1-v is the confirmation of a specific variation of the at least one analyte. Preferably the variation is length polymorphism of at least 3 bp.

Another subject-matter is a kit, preferably for use in the method for the detection of at least one or more molecular genetic analytes comprising

- the combination of probes of alternative a) as defined and described herein and
- at least one primer pair (R, F) specific for the respective target sequence (T1-v, T1-w)
- optionally enhancer as defined herein,
- optionally all components for the desired PCR technology, preferably for Modaplex, and
- optionally a reference representing T1-w, in particular a digital reference sequence or a reference nucleic acid sequence material as defined herein.

In a preferred embodiment of the method, the probe combinations a), b) and/or c), their use in the various embodiments of the method, of the hydrolysis products (F1-up/F1-down), amplicons (A1-w/A1-v), their uses and of the Kit, the respective aspect is tailored for oncological disease, more preferably for a cancer of the myeloid line of blood cells, leukemic cancers, such as Acute myeloid leukemia (AML). The feasibility of the present invention for AML detection is described in example 4.

**Definitions**

If not defined above, in the following definitions are provide for all aspects of the present invention.

An **analyte** is generically defined as a constituent of a sample with a measurable property (ISO 18113-1:2009). Molecular diagnostics of nucleic acids is a collection of techniques used to analyse nucleic acid sequence variations (e. g. genotyping of alleles like single nucleotide polymorphisms, deletion insertion polymorphisms, inversions, translocations between chromosomes, splice variants of RNA, repetitive sequences like short tandem repeats; Abbr. "VAR" in Fig. 1), epigenetic modifications (like CpG methylation or hydroxy-methylations), post-transcriptional variants (e.g. RNA editing and alternative splicing, maturation of RNA by polyadenylation, ADP-ribosylation, adenine to inosine conversion, ribonuclease degradation etc.) or quantitative alterations of defined DNA (like copy number variants) or RNA (like mRNA in gene expression) sequences. Nucleic acid sequence variants are surrounded by unique stretches of constant nucleic acid sequences which define their position within reference genomes and, thus, their specificity in complex genomes or DNA mixtures of different organisms (e. g. analysis of microbiomes, environmental specimens). Constant DNA stretches up to 35 bp, which are defined herein as **addressing sequences**, are almost sufficient (depending on the sequence complexity and peculiarities of the detection technology) to define nucleic acid primers and/or probes to analyse the presence of a unique DNA variant within a complex test sample of purified DNA. Taking into account these characteristics any nucleic acid sequence variation (synonym "variant", "molecular variation"), epigenetic modification or quantitative nucleic acid alteration together with at least one unique addressing sequence defines a specific **molecular genetic analyte (synonym "analyte")**.

A **variation** (synonym "variant" or "molecular variation"), **in particular pathologic variation**, according to the present invention, means any molecular deviation of a nucleic acid sequence (e.g. T1-v) in relation to a wildtype nucleic acid sequence of the same region that would be considered the wildtype (T1-w). A "variation" is part of the analyte as defined herein, and is surrounded by unique stretches of constant nucleic acid sequences (see above **addressing sequences**) which define their position within reference genomes and, thus, their specificity in complex genomes or DNA mixtures of different organisms. Some variations are shown in Fig. 1C and the region comprising at least one variation is abbreviated by "VAR" in Fig. 1A/B. A variation can comprise

- at least one deletion of at least one base pair (bp),
- at least one insertion of at least one base pair (bp),
- at least one deletion-insertion polymorphism (DIP), and/or
- at least one single nucleotide polymorphism (SNP) comprising transversion and transition

and/ or combinations of any of the above-mentioned types of variation. The variation may cause a diseases, such as oncologic diseases, e.g. AML, or auto-immune diseases, or be silent without causing any pathological condition. Preferably, the variation according to the present invention causes a pathological condition. More preferably the variation is specific for an oncologic diseases, e. g. AML, auto-immune diseases and/or antibiotic resistance.

The target sequence with the at least one variation (T1-v) may comprise two or more variations (T1-v1, T1-v2 to T1-$v_n$, wherein n is an integer). A continuous (not interrupted by wildtype sequence) variant region is one variation, such as a SNP, deletion or insertion of one or more adjacent bps. Wherein T1 comprise different variant region and in between the wildtype sequence, T1 can also comprise at least two variations v1 and v2 or more $v_n$. For example two SNP or two one bp deletion at different positions with a region of the wildtype sequence in between compared to the respective wildtype sequence. In combination with the amplicons - as described herein - those can be detected.

A **target sequence** (synonyms: target, **Target nucleic acid** or **template sequence or template**) is a nucleic acid sequence which includes all information that is required for the detection of a molecular genetic analyte applying a specific technology. This encompasses the molecular variant (sequence and/or quantitative variant), the addressing sequence(s) and all further sequence information which is critical for technical reasons like sequence amplification (e.g. primer binding sites in case of polymerase chain reaction, accessory primer binding sites in case of loop-mediated isothermal amplification) or selective enrichment and/or detection by probe hybridisation.

**Biomarkers** are analytes with scientific validity, whereupon 'scientific validity of an analyte' means the association of an analyte with a biological condition. Biomarkers are for example used to detect pathogens, to assess environmental or bio-process conditions, for food quality testing, in plant and animal breeding, in forensics, in veterinary and human medicine. In case of in-vitro diagnostic medical devices they act as indicators of a normal or pathogenic biological process of a human being (or patient). They also allow assessment of the pharmacological response to a therapeutic invention. A medical biomarker shows a specific physical trait or measurable biologically produced change in the body that is linked to a disease (Carini et al. 2019). The scientific validity of clinical biomarkers must be shown in extensive clinical exploration and validation trials. Furthermore, it should be mentioned that pre-analytic (e.g. patient conditioning, sampling, sample storage and transport, and test sample preparation) and analytic conditions (capabilities of the applied nucleic acid amplification and detection technology) have substantial impact on the scientific validity of the biomarkers, as well as the robustness and reproducibility of a diagnostic assay.

A **test sample** is defined as the end product of the pre-analytical process which is directly applied in the analytical reaction. It includes the analyte of interest in a mixture together with matrix chemicals, the composition and concentration of which depend on the composition of the specimen (see next definition) and the potential purification steps. Matrix chemicals may impair the analysis of the analyte or biomarker (matrix effect). The test sample includes the analyte or biomarker of interest and/or controls (water or nucleic acid free sample buffer as negative control, positive controls with spike-in reference standards, and/or internal amplification control). Test samples must be distinguished from **specimen** and **primary sample**.

Therefore, any embodiment of the method of the present invention may comprise a step of providing a test sample.

**Specimen** comprises any conceivable source materials having biological amount including an analyte or biomarker, or an artificial barcode sequence (e.g. used for traceability application). In clinical diagnostic settings specimen (also referred to as biopsy from a living body or autopsy dissected post-mortem) is a discrete portion of a body fluid or tissue taken for examination, study or analysis of one or more quantities or properties assumed to apply for the whole (ISO 18113-1:2009 and ISO 21474-1:2019).

Therefore, any embodiment of the method of the present invention may comprise a step of isolation and/or purification of the at least one target sequence T1-w and potential T1-v prior providing a test sample.

**Primary sample** (or a subsample of it) is the sample prepared from the specimen for sending to, or as received by, the laboratory and which is intended for examination (ISO 21474-1:2019).

**Oligonucleotide synthesis** is the chemical synthesis of short fragments (typical 6-120 bases with acceptable yields) of nucleic acids with defined sequences. Native nucleic acids consist of a ribose (in case of RNA) or 2-deoxyribose (in case of DNA) backbone which is bridged 5' to 3' by phosphodiesters, and the nucleotide bases adenine, cytosine, guanine, thymine, and uracil (in RNA instead of thymine) at 1'-position. Oligonucleotide synthesis is carried out by a stepwise addition of nucleotide residues to the 5'-terminus of the growing chain (3' to 5' direction) which is inverse to the enzymatic synthesis by nucleic acid template dependent nucleotidyl transferases. Currently, the preferred technology of oligonucleotide synthesis is based on solid-phase synthesis and automation using the phosphoramidite method and phosphoramidite building blocks derived from protected 2'-deoxynucleosides (dA, dC, dG, and dT), or ribonucleosides (A, C, G, and U) (Herdewijn 2005; Abramova 2013). Recently, soluble synthesis supports revive (Lönnberg 2017).

**Modifications of a (synthetic) nucleotide or oligonucleotides** comprise any chemical artificial alterations to one or more positions of the sugar backbone itself, to one or more functional groups of the backbone, to a nucleotide bases, encompasses replacement of the sugar ring and/or replacement of nucleotide bases or any combination of the aforementioned modifications, in particular as subsequently defined.

**Backbone Modifications of (synthetic) nucleotides or oligonucleotides or nucleic acid analogues as defined in this invention** (comprise artificial modifications at the 2'and/or 4' position of the five-carbon sugar (ribose or deoxyribose): The backbone of oligonucleotides refers to the internal nucleotide linkage and/or the sugar moiety. In bridged nucleic acids (BNAs) the sugar ring is modified via a bridge or third ring structure. ENA (2'-O,4'-C-ethylene-bridged nucleic acid), BNA3 (2'-O,4'-aminoethylene bridged nucleic acid), and LNA (Locked Nucleic Acid, ribose moiety modified with an extra bridge connecting the 2'-oxygen and 4'-carbon) are well established examples, but others are also available. Other neutral backbones use for example the phosphorodiamidate morpholino oligomer (PMO) or the peptide nucleic acid (PNA) modifications. Backbone modifications that are forming an internal bridge are summarized in a group of "bridging backbone modification" and can be different from those listed herein. The above backbone modifications are known to confer resistance to nucleases and may increase the duplex stability of primers and probes with target nucleic acids. Modifications of the 2'-sugar position with methyl and methoxyethyl groups are also possible. Modifications at the 2'-position are well tolerated in oligonucleotide duplexes.

**Non-backbone modifications of synthetic nucleotides or oligonucleotides** are artificial chemical modifications which are coupled to the ends (5' or 3') of an oligonucleotide or to internal nucleotide bases. This can be done during solid-phase synthesis of oligonucleotides at the 5'-end or internally using specifically modified phosphoramidite building blocks or at the 3'-end by starting the cycle oligonucleotide synthesis with specifically modified solid support materials like controlled pore glass (CPG) or macroporous polystyrene (MPPS). Alternatively, reactive chemical groups (e. g. thiol, amino, carboxyl, terminal alkyne) can be introduced during solid-phase synthesis by non-nucleoside phosphoramidites or attached by post-synthetic processing steps (after having finished the automated synthesis and cleavage from the support) to become available for a variety of chemical coupling reactions. One or two non-bridging oxygen atoms of the phosphate group can be replaced by sulfur giving rise to phorothioates (PTO) or phosphorodithioates (diPTO), respectively. In alkyl- or aryl-phosphonates which are uncharged analogues of phosphodiesters, a non-bridging oxygen atom of the phosphate group has been replaced with an alkyl or aryl group. Such none-backbone modification confer resistance, in particular of the nucleotide carrying the at least one modification, to a nuclease activity.

**Spacers** as defined in this invention are a subgroup of non-backbone modifications and are defined as chemical structures which are coupled to the 3'-and/or 5'-end of a nucleotide or between two nucleotides (Fig 9). Spacers comprise

a) alkyl alcohol of $(C_n)$-OH, wherein n is an integer and at least 3, preferably, 3, 6, 9 or 12 comprising propanyl (Spacer C3), hexanyl (Spacer C6), nonanyl (Spacer C9) and dodecanyl (Spacer C12),

b) glycol ether of $(-CH_2-O-CH_2-CH_2)_n$-OH wherein n is an integer and at least 1, preferably comprising triethylene glycol (Spacer 9), tetraethylene glycol (Spacer 12), and hexaethylene glycol (Spacer 18) and/or

c) a tetrahydrofuaran derivative containing a methylene group occupied in the 1 position of 2'-deoxyribose, also known as abasic furan, abasic spacer or dSpacer.

The above mentioned chemical structures are only some examples representing the most suitable spacers but other spacers are possible and those examples are encompassed by the above definition. The spacer is coupled during the phosphoramidite synthesis and is flanked by two phosphodiester bonds or other bringing backbone modifications. The next 5' building block can be a conventional nucleotide, a non-conventional nucleotide, an additional spacer or a linker with a fluorophore.

**Modified or artificial bases** which substitute their natural 2'-deoxynucleoside or ribonucleoside counterparts are a subgroup of non-backbone modifications which increases nucleic acid duplex stability due to internal interaction with other bases via H-bounds. Modified bases comprise 2-amino-deoxyadenosine (2-amino-dA), 5-methyl-deoxycytidine (5-Me-dC), aminoethyl-phenoxazine-deoxycytidine (AP-dC, G-Clamp), C-5 propynyl-deoxycytidine (pdC), and C-5 propynyl-deoxyuridine (pdU). In addition, intercalating nucleic acids like oTINA {ortho-twisted intercalating nucleic acid; (S)-1-O-[2-(1-pyrenylethynyl) phenylmethyl] glycerol} and (S)-1-O-(4, 4'-dimethoxytriphenylmethyl)-3-O-(1-pyrenylmethyl) glycerol intercalating pseudo-nucleotide (IPN), or 3' minor grove binder (MGB, WO1996032496A2) serves as DNA duplex stabilizers and/or polymerase blockers.

A **Quencher**, is a molecular structure being capable of accepting photoluminescence resonance energy transfer, it absorbs energy that is dissipated by a fluorophore and emits it either as light of a longer wavelength or as heat. The former is also known as fluorescence or Forster resonance energy transfer or FRET. It is important to match the absorption maximum of the specific quencher to the fluorophore on the matching probe in order to achieve efficient quenching (as in the state of the art). Tide Quencher (TQ2, biomers.net GmbH, DE, Ulm) and Iowa Black® fluorescence quencher are quencher being capable of accepting photoluminescence resonance energy transfer. Other quencher are Dabcyl (4,4-Dimethylaminoazobenzene-4-carboxylic acid), fluorescent dyes, non-fluorescent quencher (Dark Quenchers). The skilled person knows suitable quencher which e.g. available at biomers.net GmbH, DE, Ulm. Also considered as quencher according to the present invention are "up"onverting nanoparticles" as disclosed in Ding et al. 2022 which are suitable to be combined for alterative b) or c) of the probe combination according to the present invention.

**Internal nuclease blocker** (synonym internal blocker or nuclease blocker) is defined as a nucleotide having at least one artificial modification which confer resistance of said nucleotide to a nuclease activity, in particular to nucleases. In other words, certain modifications described herein fulfill the function of blocking the nuclease at the position of the modification and thereby defining the cleavage site for the nuclease being 5'upstream from the at least one internal nuclease blocker of the hybridizing sequence. The addition of a second (or more) nuclease blockers downstream from the first does not change the cleavage site but reduces unwanted artefacts in the method described herein. In the event the cleavable hydrolysis product of the invention comprises a FLAP as described herein, a nuclease blocker can be located at the FLAP and a second internal nuclease blocker is located 3' downstream from the at least one hybridizing nucleotide of the 5'-end sequence of the cleavable product as described herein (see Fig. 1A). A suitable modification is a backbone modification, more preferably, a modification at 2'-position of the sugar backbone as defined above. Most preferably theses are comprising 2'-O-methyl and 2'-O-methoxyethyl. Non-backbone modifications as defined above, preferably a modification of the phosphate group, wherein one or two non-bridging oxygen atoms of the phosphate group is/are replaced by sulfur, alkyl- and/or aryl-group confers resistance to a nuclease activity, in particular to nucleases. Finally, any artificial modification or combination of two or more of the aforementioned modifications of a nucleotide conferring resistance to a nuclease activity functions as an internal nuclease blocker within the meaning of the invention. Preferably, the internal nuclease blocker is a bridged or intercalating nucleic acid, like LNA, TINA respectively or others described herein or known in the prior art.

The at least one internal blocker has different functions. It contributes to the specificity of the signal or it contributes to the stringency of the signal without any impact on the specificity. Dependent on the label, preferably fluorophore, selected for the specific design of the cleavable product of the probe combination, in particular for alternative a) and c), within the inventive method, the achieved signal (peak) is distinct and clear (without unwanted artefacts) and therefore specific within the meaning of the invention but may be accompanied by weak or dim lower migrating artefacts depending on the label. In such cases it is preferred to combine at least two or three nuclease blockers within the cleavable hydrolysis product of the probe combination, in particular for alternative a) and c). The first will have the position as described herein and shown in Fig. 1A and the second and third may be located immediately downstream of the aforementioned position in Fig. 1A. The internal blocker also facilitates higher multiplexing, by combining it with flapped (mismatched) nucleotides upstream from the cleavage site. For specificity of the signal (see further definitions), the first flapped nucleotide, the position immediately upstream from the cleavage site, contains an internal nuclease blocker. Addition of flapped nucleotides beyond the blocked flapped position, allow stringent control of the size of the cleavable hydrolysis product and its migration. In turn, this enables an increase of the multiplex degree that the present technology, in particular for a real-time PCR with a CE, can support.

The aim of the at least one modification and/or internal nuclease blocker of the probes according to the present invention, preferably of alternative a) and/or c), is to ensure that a defined hydrolysis product is released from said probes by an enzyme exhibiting nuclease activity described herein at a defined cleavage site and is suitable for use in the method of the present invention, which is suitable to combine a plurality of said probes.

Another function of the internal nuclease blocker is the increase in the melting temperature $T_m$ of the respective sequence. This change in melting temperature is calculated by methods according to the state of the art (Natsume et al. 2007). Another function of the internal nuclease blocker is by utilizing the increase in melting temperature to increase the delta $T_m$ in the mismatch region of the downstream probe.

**Linkers** are resulting from **coupling reactions: Coupling reactions** as defined herein are any chemical reactions which are used to introduce a backbone, non-backbone modifications and/or label to the probe, in particular to a nucleotide, and which are compatible with solid-phase phosphoramidite synthesis and/or post-synthetic processing steps (e.g. release from solid support, alkaline deprotection with inorganic bases or amines, HPLC) of oligonucleotides. A subtotal selection of well-established chemistries are reactions of (a) activated organic acids [acid anhydrides, acid chlorides, N-hydroxy-succinimide (NHS)-esters] or isothiocyanates with amino groups, (b) hydrazide or aminooxy groups with aldehyde groups, (c) iodoacetamide or maleimide (2,5-pyrroledione) with thiol groups, (d) cyano-benzothiazoles with cysteine groups, (e) Click Chemistry (azide-alkyne cycloaddition; El-Sagheer and Brown, 2012) without and with metal ion catalysis (e. g. copper ions), e.g. Copper-catalysed azide alkyne cycloaddition (CuAAC), and (f) Retro- (or reverse)-Diels-Alder (rDA) reactions (e. g. reaction pairs tetrazine - trans-cyclooctene or tetrazine - norbornene). The different coupling reactions yield chemical structures which are referred to as **linkers** in this patent application and which are between the (oligo)nucleotide and its modification or between the nucleotide and the label, preferably fluorophore. Linkers which are currently available as reactive phosphoramidite regents are e.g.

2-(3-aminopropyl)-1,3-dihydroxypropane phosphoramidite
2,2-di(e-aminopropyl)-1,3-dihydroxypropane phosphoramidite
3-amino-1,2-dihydroxypropane phosphoramidite
6-amino-1,2-dihydroxyhexane phosphoramidite

Copper-catalysed azide alkyne cycloaddition - CuAAC is a type of click chemistry to link the 5' modifications with the first nucleotide of a probe/primer/oligo. A reactive 5'-alkine is achieved by coupling of an appropriate cyclohexyl-linker phopshoramidite to the oligonucleotide. It is clear to the skilled person that neither the linker or nor any other modification according to the invention does affect the activity of the DNA polymerase or of the endo or exonuclease.

A **label** comprises **fluorophore** (or **fluorescent label**) **which** are fluorescent chemical compounds that can emit light upon light excitation or any other compound which is suitable for a labeling and a detection reaction to detect the presence of a particular sequence (analyte, target sequence and/or biomarker) within a sample. The applicability of fluorophores within a diagnostic measurement device depends on the optical setting (light sources and optical filters) of its detection unit. Other compounds comprise biotin, digoxigenin, haptens etc. Usually labels are coupled via a linker to the 5'phosphat group of a nucleotide. Alternatively, the 2'-position has been widely used for the attachment of fluorophores

The Modaplex device (Hlousek et al. 2012, synonym: Modaplex), as used within this invention, is a closed system for real-time multiplex nucleic acid analysis combining two methods i) polymerase chain reaction (PCR) and simultaneous ii) capillary electrophoresis (CE). During a Modaplex run, the genetic targets of interest are amplified in a PCR reaction. While the PCR reaction continues undisturbed, the amplified DNA fragments are simultaneously electrokinetically injected into a capillary gel. In this gel, the DNA fragments are separated by size and quantitatively detected. This process is repeated after each PCR cycle. In this way, real-time data is generated, thus enabling the simultaneous quantification of up to 50 targets. Modaplex device detects fluorescence signals in two different optical channels 1 (blue) and 2 (red) with excitation wavelengths 488 nm and 636+/-8nm, and emission filters of 535+/-35 nm and 675+/-25 nm, respectively. The two channels are originally calibrated by the emission of the fluorophores FAM/fluorescein (emission maximum at 520 nm) and TYE665 (emission maximum at 665 nm; property of Integrated DNA Technologies Inc, Coralville, US-IA). Examples of alternative fluorophores for channel 1 and channel 2 of the Modaplex device are shown below:

| channel 1 (blue channel) | channel 2 (red channel) |
| --- | --- |
| Atto465, Cyanine 2, Dy-478, Oregon Green 488, Dy-488, Dy-490, Alexa Fluor 488, (5), 6-FAM/Fluorescein/ FITC, Dy-495, Atto488, Abbrerior STAR 488, Alexa Flour 500, Atto 495, Dy-505 | AttoRho14, Abberior STAR 635, Abberior STAR RED, Atto633, Dy-630, Dy-631, Dy-632, Dy-633, Dy-634, Dy-635, Dy-641, Atto647N, Atto643, Alexa Fluor 647, Quasar 670, Cyanine 5, TYE665, LC Red 670, Dy-636, Dy-647, Dy-647P1, Dy-648, Dy-648P1, Dy-649, Dy-649P1, Dy-650, SiR, SPY650, Dy-651, Dy-652, Dy-654, LIZ, LC Red 670, |

However, other fluorophores and combinations for channels 1 and 2 are possible. Other cyanines comprise CY3, CY3.5, CY5, CY5.5, CY7; rhodamine and derivatives such as Texas Red, R6G, R110, TAMRA, ROX; fluorescein and derivatives such as 5-bromomethyl fluorescein, 2',7'-dimethoxy-4',5'-dichloro-6-carboxyrhodamine (JOE), 6-carboxylfluorescein (6-FAM), 1,2',4',1,4,-tetra chlorofluorescein (TET), 2',4',5',7',1,4-hexa chlorofluorescein (HEX), Lucifer Yellow, IAEDANS, benzophenoxazines, 7-Me 2N-coumarin-4-acetate, 7-OH-4-CH 3-coumarin-3-acetate, 7-NH 2-4-CH 3-coumarin-3-acetate (AMCA), monobromobimane, pyrene trisulfonates, such as Cascade Blue, Oregon Green, and monobromorimethyl-ammoniobimane. Additional examples of fluorescent dyes are provided in, e.g., Johnson I and Spent MY (2010), and the updates thereto, which are each incorporated by reference. Fluorescent dyes are generally readily available from various commercial suppliers including, e.g., Molecular Probes, Inc. (Eugene, US-OR) Amersham Biosciences Corp. (Piscataway, US-NJ), Atto-Tec GmbH (Siegen, DE), Dyomics GmbH (Jena, DE), Applied Biosystems (Foster City, US-CA) etc. Fluorescein dyes and derivatives thereof are particularly preferred in the methods described herein. The appropriate design of the probes according to the present invention, preferably of alternative a) and/or c), comprise a suitable label, preferably fluorophore, that enables the use of the full range of the electropherogram for each fluorescence channel. The range of the electropherogram that can be achieved by the use of probes of the present invention, preferably of alternative a) and/or c), depends on the detection range of the used CE device. The range for Modaplex device used herein is up to 450 bp but the technology described herein is also applicable to CE devices, preferably Modaplex devices, with ranges above 500 bp. For these embodiments the probes of alt. a) or c) and combination of modifications described herein can be designed accordingly. In the examples the probe of alternative a) is compared with the probe of alternative b).

**FEN enhancer oligonucleotide (synonym: (FEN) enhancer)** is a nucleotide sequence structure which increases the intrinsic FEN activity of a polymerase, preferably of the Taq DNA polymerase. The addition of at least one FEN enhancer oligonucleotide (ENH1-n) increases the intrinsic FEN activity of a polymerase, preferably of the Taq DNA polymerase, at least quantitatively and optionally qualitatively. In ENH1-n n is an integer. Preferably greater than or equal to 1 to less than or equal to 50. In particular, n is an integer and preferably equal to 2, 3, 4, 5, 6, 7, 8, 9 or 10. The at least one FEN enhancer oligonucleotide (ENH1-n) may be labeled or not. A FEN enhancer oligonucleotide (ENH$_{1-n}$) hybridizes with the target sequence immediately upstream of the upstream probe. In this case, the 3'-end of the FEN enhancer oligonucleotide (ENH$_{1-n}$) overlaps with sequence region (1) of the respective probe, which is paired with the target sequence exactly to the double helix, by at least one nucleotide (See Fig. 1 of WO2017/046191A1). The 3'-sequence of the FEN enhancer oligonucleotide overlapping with the probe does not necessarily have to thereby hybridize with the target sequence but may form an unpaired 3'-flap. This arrangement results in a significant increase in cleavage activity of the intrinsic FEN of a polymerase, preferable of the Taq DNA polymerase. Other structural properties of FEN enhancer oligonucleotides are conceivable for other FEN enzymes. The detailed design of said enhancers and function are disclosed in WO2017/046191A1, wherein it was shown that the at least one enhancer results an at least 5-fold enhancement of the signal of the at least one obtained amplicon (see example 1 and 3 of WO2017/046191A1). According to the present invention the probe combination of alt. a) and/or c) - as defined herein - can be combined with at least one enhancer and can be used in said combination in the method according to the present invention, preferably within a quantitative and preferably continuous real-time PCR with capillary electrophorese, most preferably within the Modaplex device (Biotype GmbH, Dresden).

**Nucleic acid amplification technologies** (NAT) refer to enzymatic methods for in-vitro duplication of nucleic acids, in particularly, of target sequences according to the invention. According to the invention, the target sequence is amplified in a repeated duplication reaction, preferably in a duplication reaction of a polymerase chain reaction (PCR), which requires thermic cycles. PCR is well known by the person skilled in the art. RNA can be amplified after conversion into complimentary DNA (cDNA) by a reverse transcriptase. EP0506889B1, EP0632134B1, EP1152062B1, WO2014023318A1 disclose furthermore different technologies for real-time reverse transcriptase PCR to amplify RNA in single reaction tubes. Other NAT proceed isothermally (iNAT). LAMP (loop-mediated isothermal amplification), HDA (helicase-dependent amplification), RPA (recombinase polymerase amplification), SIBA (Strand Invasion Based Amplification), RCA (rolling circle amplification) are examples for the isothermal amplification of DNA. Finally, Real-Time NASBA (nucleic acids sequence-based amplification) describes an iNAT for the direct amplification of RNA in combination with a hydrolysis probe (Keigthley et al. 2005). The expert in the field knows of other technologies such as NGS that may be based on NAT.

**Multiplex NAT or in general multiplex tests** (synonym multiplexing approach) describe the ability of an assay to interrogate or detect more than one analyte or biomarker simultaneously in each test sample, ideally within one reaction. Multiplex assays provide time, cost and information content advantages, and therefore allow for higher confidence results than singleplex assays ("monoplex" and "singleplex" are synonyms). In addition, the less hand-on steps reduce the risk of sample mix-up or cross-contamination. Preferably "multiplex" (synonym multiplexing) refers to the detection of multiple different analyte, preferably within different target sequences of at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 30 or more targets, e.g., at least 50, at least 100, at least 250 or more targets. Preferably at least 20, more preferably at least 30.

**Nucleic acid electrophoresis** as defined herein are technologies to separate nucleic acid amplification products by the combination of mass and charge within an electric field. Negatively charged nucleic acid fragments are applied on the cathodal side of a pair of electrodes which are connected by an aqueous buffer. The separation of molecules is improved by sieving polymers like agarose or acrylamides (cross-linked or linear, e.g. Performance Optimized Polymer POP™-7, Thermo Fisher Scientific Inc., Waltham, US-MA) with jellylike consistency. Flat gel and capillary gel electrophoretic (CE) devices can be distinguished regarding the setting of the gel porter, whereas CE devices are more convenient for automation. Furthermore, PCR amplification products can be separated as double stranded DNA (dsDNA) applying so called native conditions or as single strands (ssDNA) in the presence of denaturing agents like urea or heat or combinations thereof.

The Modaplex device (Hlousek et al. 2012) which has been used within this invention is an automated system that combines a PCR thermocycler together a denaturing capillary electrophoresis (CE) system to allow multiplex amplification of DNA sequence targets and the quantification of DNA analytes or biomarkers, simultaneously. Other examples of CE devices which also support the invention and which have different optical detection units (see definition of fluorophores for MODAPLEX detector characteristics) with one to six channels are Applied Biosystems 3500/3500xL Genetic Analyzers and Applied Biosystems SeqStudio instruments (Thermo Fisher Scientific Inc., Waltham, US-MA), Applied Bioystems RapidHIT ID System, Spectrum CE Systems (Promega Corp., Madison, US-WI), Beckmann Coulter CEQ™ 8000 Genetic Analysis System (Fullerton, US-CA), QIAxcel Advanced System (Qiagen GmbH, Hilden, DE), Nanofor® 05M (Syntol, Moscow, RU), CEQ8800 DNA Sequencer (Beckman Coulter, Brea, US-CA)and diverse nucleic acid fragment analyzers from Agilent Technologies Inc. (Santa Clara, US-CA) like TapeStation, Bioanalyzer, Fragment Analyzer System, ZAG DNA Analyzer System, Femto Pulse System.

As used herein, **MODAPLEX calibrators** are at least one, preferably two or more non-amplifiable size standards detected in capillary electrophoresis in at least one fluorescence channel in a multiplex setup, which are unrelated to the analytes or biomarkers of interest (unrelated or artificial templates and primers). They are comprised by short DNA fragments, 1-5 nucleotides in length, with a fluorophore attached at the 5'end but also can be attached to an internal nucleotide. They define the range of amplicon detection by the analyzing software of the MODAPLEX instrument. In the described examples of this invention **MODAPLEX size standard Mix** (Biotype GmbH, DE) was used in a onefold final concentration. It consists of 5 size standards whose signal intensity is constant through PCR cycling. An amplification control (AC) calibrator consists of artificial templates (unrelated to human DNA), the corresponding PCR primer pairs and the corresponding probes of alt. a) or c). The AC plus 3 non-amplifying size standards run in the 6-FAM channel (blue channel) and 2 calibrators run in TYE665 fluorescence channel (red channel). The calculated length sizes were 114.5 bp, 169.18 bp, and 277.5 bp on the blue channel and 150.7 bp and 440.4 bp on the red channel. The AC's calculated length is 58.6 bp. However, the apparent length can slightly differ by a maximum of 5 bp without impact on assay analysis. The AC calibrator also represents a template-independent PCR control and should be added to all sample, negative and positive control wells. Alternatively, the PCR setup can be spiked by calibrators in an appropriate endpoint concentration. In this case other markers must be used as a template-independent PCR control. The regions between the MODAPLEX calibrators can be subdivided into regions for target, allele or profile calling of the amplicons of interest. However, an exact amplicon size calling is not possible.

**Migration Time** is herein defined as the time that takes a labelled analyte, it being, according to the invention, labelled nucleic acids, to reach the detection point after electrokinetic injection at each PCR cycle. The MODAPLEX uses "scans" as a unit for migration time, which corresponds to around 150 ms, due to the scanning rate of the detector of around 6.66Hz. Migration times can be converted to migration lengths by doing a linear fit relating the known migration lengths of the calibrator system components with their respective migration times (see above the definition of MODAPLEX calibrator).

**FLAP endonucleases** (FEN) are structure- and strand-specific endonucleases which cleave the single-stranded DNA- or RNA-sequence of a fork-shaped unpaired 5'-end (5'-FLAP) of a DNA double helix (Lyamichev et al. 1993).

The nuclease preferably cleaves after the first 5'-hybridized nucleotide but is not restricted to this cleavage site. It can also cleave after second, third, fourth and fifth hybridized nucleotide of 5'-flapped nucleotides. Thus, the hydrolysis reaction mainly results in hydrolysis products with the size n+1 but also n+2, n+3, n+4, n+5 and n, n-1, n-2, n-3, n-4, etc., whereas the minimum fragment size in one. By combination of the modifications described herein the desired cleavage site as defined herein and thereby the activity of the nuclease can be determined and directed. FEN occur in all living organisms and even virus (Mitsunobu et al. 2014) and release in conjunction with further enzymes, in particular during DNA replication, the so-called Okazaki fragments (RNA-DNA hybrids) at the remaining strand of the replication fork (DNA repair function). Eubacterial FEN form in combination with a DNA polymerase of type Pol 1 (synonymous = Pol A) a single protein unit (e.g. Pol 1 of Escherichia coli, Thermus aquaticus, T. thermophilus, Aquifex spp.). Archaebacterial (e.g. Archaeoglobus fulgidus, Pyrococcus spp., Methanocaldococcus jannaschii, Methano-thermobacter thermoautotrophicum), so far described virus encoded and eukaryotic FEN (e.g. Homo sapiens) represent autonomous proteins. Preferentially, thermostable enzymes are used herein.

**Hydrolysis (cleavage) product.** At least one 5'-terminal nucleotide of a completely to the target sequence hybridized oligonucleotide which is hydrolyzed by a nuclease, preferably the FEN activity of a DNA-dependent DNA polymerase (e.g. Taq DNA polymerase). The resulting hydrolysis fragment (synonym for "hydrolysis (cleavage) product") always consists of at least one nucleotide (conventional or non-conventional) with a 3'-OH group and a fluorophore coupled via a linker. Additionally, it can contain further modifications which can also include additional nucleotides if they did not hybridize to the target DNA strand (flapped nucleotides). Prior to hydrolysis by the FEN, the "hydrolysis (cleavage) product" or hydrolysis fragment is part of the probes of alt. a) or c) as defined below. It is hydrolyzed, cleaved or released from the probes of alt. a) or c) after said probes hybridized or annealed to an DNA strand of a target nucleic acid. In a preferred embodiment of the invention described herein, each hydrolysis product exhibits or is characterized by a unique migration pattern resulting a peak (curve) upon separation during a, preferably capillary, electrophoresis, as defined herein.

**Specificity of the signal**, according to the present invention, means that, for each released hydrolysis product from the respective probes of alt. a) or c) that is hybridized to the respective target sequence, only one clear and distinct peak (curve) is achieved in a capillary electrophoreses and represented in an electropherogram. An electropherogram is a record or visualization of the hydrolysed and separated hydrolysis products represented by peaks and curves that are produced when electrophoresis is used in the method of the present invention. Preferably, the clear and specific peak is a curve (see e.g. Fig. 4 or 5 or 7) as achieved by means of a CE device, such as the Modaplex device. The specificity of the signal achieved by the method described herein and by use of the released hydrolysis products from the inventive probes of alt. a) or c) described herein, is achieved due to modifications and the nuclease blocker described herein. In a preferred embodiment a "clear and specific signal" means that only the hydrolysis products without unwanted artefacts are detected and that those unwanted artefacts do not occur. Dependent on the target sequence, and in particular dependent on the nucleotide at the 3'-end of the target sequence to that the 5'-end of the cleavable hydrolysis product of the probes of alt. a) or c) hybridize, one nuclease blocker and/or modification may be not sufficient to achieve a specific signal. Thus, for such target sequences the cleavable products of the probes of alt. a) or c) comprise at least two, three or more, preferably two or three, nuclease blocker and/or modification, in order to ensure a specific signal according to the present invention. One suitable solution for such target sequences that enables a specific signal is a first nuclease blocker at the second 5'-end nucleotide and a second nuclease blocker at the third hybridizing nucleotide of the 5'-end sequence of the cleavable hydrolysis product of the probes of alt. a) or c)), wherein said cleavable hydrolysis products and probes of alt. a) or c) are fully hybridized to its complementary sequence on the target sequences. In another embodiment, wherein the cleavable hydrolysis product comprises a FLAP as described herein, the first nuclease blocker is located at the FLAP and a second nuclease blocker is located 3'upstream from the at least one hybridizing nucleotide of the 5'-end sequence of the cleavable product as described herein (see Fig. 1A), e.g. the first nuclease blocker is at the one non-hybridizing nucleotide being the FLAP (=first non-hybridizing nucleotide of cleavable hydrolysis product) and the second and internal nuclease blocker is at the third hybridizing nucleotide of cleavable hydrolysis product (see Fig. 1B).

Oligonucleotide probes ("probe", upstream or downstream probe see also Fig. 1 D of alt. a) or c) comprise an oligonucleotide (preferably with a minimum length of 5 nucleotides), whose sequence specifically hybridizes to a complementary nucleic acid, preferably DNA, strand of a target nucleic acid (Fig. 1. (2)) and further comprise:

- A PCR blocker at its 3'-end (synonym protective group), as described herein
- Optionally an internal nuclease blocker for alternative a) of the probe combination as describe herein, and
- a (cleavable) **hydrolysis product** (Fig. 1, (3)) to be released by a nuclease, preferably FEN, as described herein, the cleavable **hydrolysis product** is located at the 5'-end upstream of the internal nuclease blocker for alternative a) of the probe combination and comprises

  - at its 5'-end at least one target specific (complementary) nucleotide (which is complementary to the strand of the target sequence to which) linked to the internal blocker via a phosphodiester bond.

- it may additionally comprise at least one modification, described herein, which does not hybridize to the target sequence and/or
- Optionally/preferably a label, preferably a fluorophore (e.g. for use with the herein described Modaplex Device or Applied Biosystem of Thermo Fisher Scientific), bridged via a linker to the cleavable hydrolysis product, either to the at least one modification or to the at least one target specific (complementary) nucleotide, the label can be quantified by a detecting unit of a nucleic acid electrophoresis device, preferably Modaplex device. In one embodiment of the invention the cleavable hydrolysis product does not comprise a label (e.g. for use with CE/MS Systems of Agilent) but remains detectable and quantifiable. In this embodiment the gel comprises an intercalating dye, e.g. SYBR Green.

The function of the inventive probes is to enable the detection of at least one analyte with at least one variation (T1-v) in a test sample and to provide a cleavable hydrolysis product for the generation of a specific signal. Thus, the released hydrolysis product confirms that the respective probes hybridized specifically to its complementary sequence on the target and allow the visualization of the specific hybridization trough the migration pattern with peaks (curves) of said hydrolysis products.

**Nucleic acid hybridization** (syn. hybridization) is defined as the annealing of two complementary single-stranded deoxyribonucleic acid molecules to an anti-parallel doublestranded DNA sequence (non-covalent DNA double helix formation). The two stands are mostly stabilized by hydrogen bonds between corresponding nucleic acid bases (Watson-Crick), London dispersion forces, and the hydrophobic stacking of neighboring base pairs (Altun et al. 2021). The thermodynamics of the process has been extensively studied and the most convenient sequence specific prediction method is based on the nearest-neighbor (NN) model (SantaLucia 1998). Different parameters must be considered for synthetic nucleic acid base (e.g. C5-propynyl derivatives of pyrimidine bases, He and Seela 2002) or backbone modifications like LNA (McTigue et al. 2002). Some of the aforementioned methods are considered in the software as mentioned herein i.e. the hybridization of the least one primer or of both of the primer pair in order to generate an amplicon from the target sequences T1- and T1-w according to the invention (A1-v and A1-w). However, these software cannot perform calculations for competing probes as used herein, particularly those which can discriminate mutational hotspots.

An **overlapping region** is an identical nucleic acid sequence contained in at least two different nucleic acid sequences, such as of two probes, i.e. region (2) of the upstream and downstream oligonucleotide according to the present invention.

The hybridization of a probe to their respective complimentary sequence on a target is generally assumed to be in chemical **equilibrium** between the dissociated and hybridized state of the complementary sequences (probe and target) to each other. In typical PCR conditions during the hybridization (annealing) step, this chemical equilibrium reaches a high percentage of the hybridized probe with a sequence complimentary to the target and its target sequences. When combining more than one probe with an overlapping region (see region (1) in Fig. 1.) to the respective target, there is a shift in the equilibrium due to a competition of at least two oligonucleotides for the same hybridization sequence.

In the case of a bulk PCR - in contrast to a partition as used for dPCR with a single target molecule - with a large amount of target sequences a new hybridization equilibrium will form between the three types of nucleic acids.

The **hybridization rate** for a given probe X is therefore defined as the ratio of X hybridized to the respective target sequence vs. the X non-hybridized. In the case of a hybridization equilibrium between at least two competing probes, each of these probes will have their own hybridization rate depending on many factors, such as the individual probe and target concentrations, the binding affinity of the probe, the presence or absence of mismatches to the target sequence and general reaction conditions. In the case of competing probes that are labeled differently, the hybridization rate of both probes can be indirectly inferred from the ratio of the two signals in relation to each other.

When all other reaction conditions are consistent, a shift in the signal ratio indicates a shift in the hybridization rate which might be expected in the presence of mismatches on the target sequence.

In one example of two probes competing in an overlapping region - as described herein region (2) - and a perfect match of both probes of the present invention to the target sequence T1-w, the hybridization rate of the downstream probe will be relatively high, leading to high F1-down and relatively low F1-up.

In a second example, using the reaction mix in example 1 with a target T1-v, the hybridization rate of the downstream probe will decrease, resulting in lower F1-Down, while the hybridization rate of the upstream probe due to the variation uninfluencing hybridization of region (2) and region (1), will increase along with the readout F1-up. This leads to an exchange of signal intensities between the upstream and downstream hydrolysis products.

**Description of the drawings**

**Fig. 1: Concept and probe design**

A: Schematic depiction of the principle behind the signal exchange that is caused by a lower affinity of the downstream probe to templates with sequence variations.

B: strategic placement of the downstream probe containing an overlapping sequence region (2) and a 3' region (3) in relation to the variation (VAR) that can have any size but at least 1 nucleotide (nt), corresponding to a sequence region 3.1 on the downstream probe, which is part of region (3).

C: Magnified detail of sequence region 3.1 illustrating the perfect match of downstream probe on T1-w and two versions of T1-v with different variations. To increase specificity, the LNA (f) would be strategically placed at the nucleotide of the SNP's.

D: Structure of the probes according to alternative a) with a fluorophore *a*, linker *b*, spacer *c*, the hybridizing sequence *d*, a modification with amplification blocking function *e* and the locked nucleic acid *f* close to the 5' end of the hybridizing sequence *d*. In alternative b) the quencher *g* is added to the 3' end. An alternative c) contain the required combination of elements necessary to use these probes in both technologies such as a fluorophore *a*, linker *b*, spacer *c*, the hybridizing sequence *d*, the quencher *g* and the locked nucleic acid *f* close to the 5' end of the hybridizing sequence *d*.

**Fig 2: Comparison of the probe alternative b) and probe design according to prior art with droplet digital PCR method.** The probe alternative b) and probes according to prior art (PA) were used to analyze artificial samples containing 100 % wild-type, 100 % variant and 0.5 % variant in 99.5 % wild-type form of an exon 19 EGFR target with ddPCR. The scatter plots show the FAM and Hex signals for individual droplets as a technical overlay of individual experiments. Threshold lines were set to clearly distinguish wild-type and mutant DNA. Clusters that were not properly classified with the threshold lines were reclassified with the lasso tool (dashed circle). Clusters of droplets containing one of the variant forms ("9/2/1 (nt deletion) and S(NP)") and only the wild-type form ("VV"), are identified in the plot.

**Fig 3: Comparison of the probe alternative b) and probe design according to prior art with droplet digital PCR method with low degree of variation samples.** The same experimental setup as in Fig. 2 but plots of individual samples containing 0.5% variation of only one type of mutation as indicated above each panel.

**Fig 4: Effect of LNA comprising probes in contrast to prior art) on CE results on the Modaplex™ instrument.** The probe designs according to prior art without an LNA as internal blocker were hydrolyzed generating several short fluorophore-labeled fragments in the presence of either 100 % wild-type or 100 % variant analytes corresponding to different nucleotide hydrolysis products. Peaks of interest are labeled with probe or competitor, while undesired side peaks are labeled with an asterisk. Probes with an LNA show a distinct single peak with drastically reduced side peaks.

**Fig. 5: Comparison of the probe alternative a) to probe oligo design according to prior art on the Modaplex™ instrument.** The probe alternative a) and probes according to prior art were used to analyze artificial samples containing 100 % wild-type, 100 % mutant and 5 % mutant in 95 % wild-type form of an exon 19 EGFR target on the Modaplex™ instrument. Endpoint electropherograms of all signals acquired on the blue-channel after 39 cycles are shown and peaks of interest are labeled. Peaks SST1 and SST2 correspond to non-amplifying internal size standards that are used to calculate migration lengths of hydrolysis fragments.

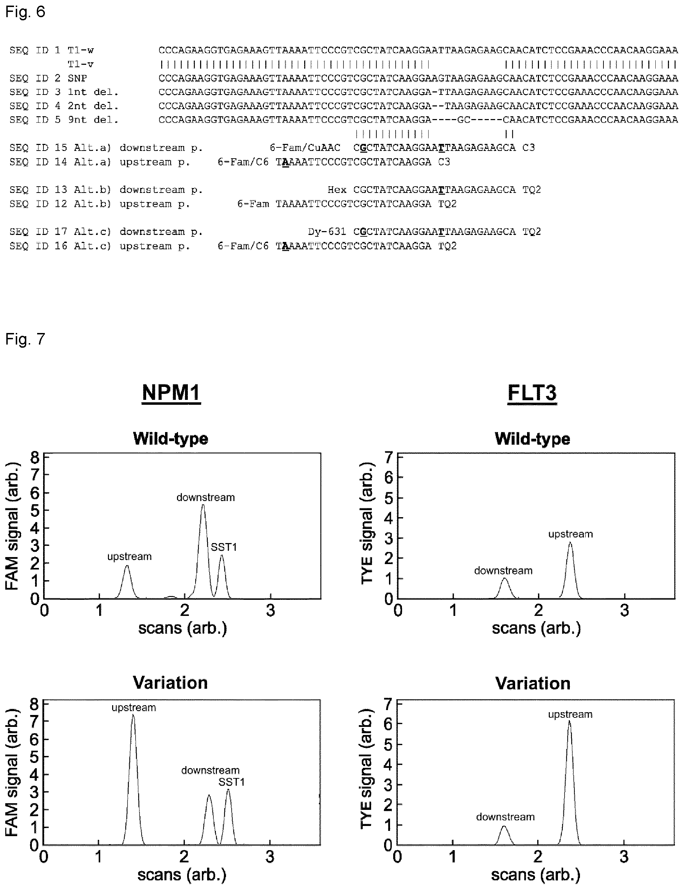

**Fig. 6 Arrangement/sequences of probes in relation to the target sequences of the examples 1 to 3** T1-w corresponds to Seq ID No. 1, T1-v comprises 4 types of variation corresponding to the indicated Seq ID Numbers: "SNP" represents a T>G transversion, "1nt del." represents the deletion of A in relation to the sequence "T1-w" in Position 43; "2nt del." represents the deletion of two nucleotides A and T in relation to the sequence "T1-w" in Position 43-44; "9nt del." represents the deletion of 11 consecutive nucleotides from position 43 and the insertion of GC in relation to the corresponding region in the sequence "T1-w"; "Alt. a) downstream p." and "Alt. a) upstream p." correspond to Seq ID No. 14 and No. 15 and are probes of alternative a) according the present invention; "Alt. b) downstream p." and "Alt. b) upstream p." correspond to Seq ID No. 13 and No. 12 and are probes of alternative b) according the present invention; Alt. c) downstream p." and "Alt. c) upstream p." correspond to Seq ID No. 17 and No. 16 and are probes of alternative c) according the present invention. All probes are depicted with their respective 5' fluorescent label and 3' modification, as well as the internal blockers in the form of LNAs (underlined and bold).

**Fig. 7 Application example of multiplex PCR reaction for NPM1 and FLT3 gene in context of acute myeloid**

**leukemia (AML) diagnostic approach.** A blood-derived DNA without variation in the tested analytes was used as wild-type reference (see example 4). For FLT3 TKD an artificial template was tested with FLT3 c.2504A>C (p.D835A) SNP variation. The OCI-AML3 cell line (DSMZ no.: ACC582) with a NPM1 type A variation was tested for NPM1. Peak SST1 corresponds to internal size standards that are used to calculate migration lengths of hydrolysis fragments. The upstream and downstream probes are probes of alternative a). For both application examples NPM1 and FLT3 show an increase of upstream signal and decrease of downstream signal on the variation compared to wild-type.

**List of references**

Altun et al. 2021 "Unveiling the complex pattern of intermolecular interactions responsible for the stability of the DNA duplex". Chem. Sci., 2021, 12, 12785 doi: 10.1039/d1sc03868k.

Carini C, Fidock M, van Gool A (eds.) (2019). Handbook of Biomarkers and Precision Medicine, CRC Press, Boca Raton, US-FL.

Ding et al. 2022 "Novel Fluorescent Probe Based on Rare-Earth Doped Upconversion Nanomaterials and Its Applications in Early Cancer Detection" *Nanomaterials* 2022, *12*(11), 1787; doi.10.3390/nano12111787.

Döhner et al. 2022 "Diagnosis and Management of AML in Adults: 2022 ELN Recommendations from an International Expert Panel." Blood (2022) 140 (12): 1345-1377. https://doi.org/10.1182/blood.2022016867.

He and Seela 2002 "Propynyl groups in duplex DNA: stability of base pairs incorporating 7-substituted 8-aza-7-deazapurines or 5-substituted pyrimidines" Nucleic Acids Research, Volume 30, Issue 24, 15 December 2002, Pages 5485-5496, doi.10.1093/nar/gkf689.

Hlousek et al. 2012 "Automated high multiplex qPCR platform for simultaneous detection and quantification of multiple nucleic acid targets." Biotechniques.52(5):316-24. doi: 10.2144/0000113852.

Johnson I, Spent MY (eds.) 2010 The Molecular Probes® handbook. A guide to fluorescent probes and labeling technologies. 11th edition. Invitrogen-Molecular Probes®, Thermo Fisher Scientific Inc., Waltham, US-MA.

Keightley et al. 2005 "Real-time NASBA detection of SARS-associated coronavirus and comparison with real-time reverse transcription-PCR". J Med Virol. 77(4):602-8. doi: 10.1002/jmv.20498.

Lyamichev et al. 1993. "Structure-specific endonucleolytic cleavage of nucleic acids by eubacterial DNA polymerases" Science. 7; 260(5109):778-83. doi: 10.1126/science.7683443. Mitsunobu et al. 2014 "Flap FLAP endonuclease activity of gene 6 exonuclease of bacteriophage T7" J Biol Chem 289: 5860-75. doi: 10.1074/jbc.M113.538611.

McTgue et al. 2004 "Sequence-Dependent Thermodynamic Parameters for Locked Nucleic Acid (LNA)-DNA Duplex Formation" Biochemistry 2004, 43, 18, 5388-5405; doi:10.1021/bi035976d.

Natsume et al. 2007 "Effect of base mismatch on the electronic properties of DNA-DNA and LNA-DNA double strands: density-functional theoretical calculations." Chem. Phys. Lett. 2007;446:151-158, doi:10.1016/j.cplett.2007.07.095.

Owczarzy et al. 2011 "Stability and mismatch discrimination of locked nucleic acid-DNA duplexes" Biochemistry 50: 9352-9367, doi10.1021/bi200904e.

SantaLucia etal. 1998 "A unified view of polymer, dumbbell, and oligonucleotide DNA nearest-neighbor thermodynamics" Proc. Natl. Acad. Sci. USA, 95 (4) 1460-1465; doi.10.1073/pnas.95.4.1460.

Tan et al. 2022 "Current commercial dPCR platforms: technology and market review. Crit Rev Biotechnol. 2022 Mar 15:1-32, doi: 10.1080/07388551.2022.2037503.

**Examples**

**Material and methods**

**Design and synthesis of PCR primers, probes and gBlocks:** Target sequences for exon 19 of the epidermal growth factor receptor (EGFR) were selected as depicted as Seq ID No. 1-5 in Figure 6. PCR primers and probes (Table 2) were designed for probe design according to the prior art as depicted as Seq ID No. 6-11, which briefly teaches using a combination of a competitor (comparable to the downstream probe that may or may not be labeled) with a much higher Tm compared to the labeled probe (comparable to the upstream probe). The designs for the probes of the present invention of alternative a), b) and c) are depicted as Seq ID No. 12-17 were based on the respective design rules explained in table 1 using the software Geneious 10.2.6 (Biomatters Ltd., Auckland, New Zealand). For the transfer of a probe design according to the teaching of the prior art (Seq ID No. 8-9) from ddPCR to the Modaplex instrument (Seq ID No. 10-11), the following adjustments had to be made: (i) probe and competitor were labeled with the same fluorophore but different linkers based on the design rules of the present invention (table 1), (ii) the quencher is necessary for ddPCR only but for Modaplex it was replaced with a Spacer C3 because it was cheaper and (iii) a LNA was inserted at the 2nd position from the 5' end based on the inventive alternative a) probe design to avoid undesired hydrolysis products. The following fluorescent labels are used in the given examples: 6-carboxylfluorescein (6-FAM); 2',4',5',7',1,4-hexa chlorofluorescein (HEX); Dy-631. For alternative a) different linkers were used in combination with the fluorophores to enable separation in qPCR in addition with CE for e.g. Modaplex: C6/hexanyl, click chemistry via 5' linker/ CuAAC (Biomers). As a blocker at the 3' end of probes according to alternative a) we have used Spacer C3 and for probes according to alternative b) and digital PCR probes according to prior art we used the Tide Quencher TQ2.

Primers were obtained in cartridge-purified quality and probes in HPLC-purified quality from biomers.net GmbH (Ulm, Germany). gBlocks were used as artificial template and obtained from Integrated DNA Technologies (IDT) Inc. (Coralville, IA, USA)].

**Table 2: Primers, probes, and artificial sequence. The primer binding temperature (Tm) was calculated using the software Geneious 10.2.6.**

| Seq ID No | Organism | Name | sequence (5' → 3'-end) | length | Loca-tion | Gene/Feature/ modification | $T_a$ [°C] |
|---|---|---|---|---|---|---|---|
| 1 | Homo Sapiens | T1-w | CTCTCTGTCA TAGGGACTCT GGATCCCAGA AGGTGAGAAA GTTAAAATTC CCGTCGCTAT CAAGGAATTA AGAGAAGCAA CATCTCCGAA ACCCAACAAG GAAATCCTCG ATGTGAGTTT CTGCTTTGCT GTGTGGGGGT CCATGGCTC | 149 | % | Wildtype EGFR (COSG150) | N/A |
| 2 | Artificial | T1-vSNP | CTCTCTGTCA TAGGGACTCT GGATCCCAGA AGGTGAGAAA GTTAAAATTC CCGTCGCTAT CAAGGAA**G**TA AGAGAAGCAA CATCTCCGAA ACCCAACAAG GAAATCCTCG ATGTGAGTTT CTGCTTTGCT GTGTGGGGGT CCATGGCTC | 149 | 68 | T>G | N/A |
| 3 | Artificial | T1-v/DEL1 | CTCTCTGTCA TAGGGACTCT GGATCCCAGA AGGTGAGAAA GTTAAAATTC CCGTCGCTAT CAAGGA-TTA AGAGAAGCAA CATCTCCGAA ACCCAACAAG GAAATCCTCG ATGTGAGTTT CTGCTTTGCT GTGTGGGGGT CCATGGCTC | 148 | 67 | Del 1nt | N/A |
| 4 | Artificial | T1-v/DEL2 | CTCTCTGTCA TAGGGACTCT GGATCCCAGA AGGTGAGAAA GTTAAAATTC CCGTCGCTAT CAAGGA--TA AGAGAAGCAA CATCTCCGAA ACCCAACAAG GAAATCCTCG ATGTGAGTTT CTGCTTTGCT GTGTGGGGGT CCATGGCTC | 147 | 67-68 | Del 2nt | N/A |
| 5 | Homo Sapiens | T1-v/DEL9 | CTCTCTGTCA TAGGGACTCT GGATCCCAGA AGGTGAGAAA GTTAAAATTC CCGTCGCTAT CAAGGA---- **GC**-----CAA CATCTCCGAA ACCCAACAAG GAAATCCTCG ATGTGAGTTT CTGCTTTGCT GTGTGGGGGT CCATGGCTC | 140 | 67-77: 76-77: | Del 11nt ins GC EGFR COSM12422 (GRCh38, 7: 55174775..55174785) | N/A |
| 6 | Artificial | Primer F | GGATCCCAGA AGGTGAG | 17 | 1-17 on T1-w | | 58,4 |

(continued)

| Seq ID No | Organism | Name | sequence (5' → 3'-end) | length | Loca-tion | Gene/Feature/ modification | $T_a$ [°C] |
|---|---|---|---|---|---|---|---|
| 7 | Artificial | Pimer R | ATCGAGGATT TCCTTGTTG | 19 | 130-149 on T1-w | | 58 |
| 8 | Artificial | Probe Prior Art $\Delta T_m > 6$ in dPCR | 6-FAM AATTCCCGTC GCTATCAA TQ2 | 18 | (18) | Quencher TQ2 | 59,2 |
| 9 | Artificial | competitor Prior Art $\Delta T_m > 6$ in dPCR | HEX CGTCGCTATC AAGGAATTAA GAGAAGCA TQ2 | 29 | (29) | Quencher TQ2 | 66,7 |
| 10 | Artificial | Probe Prior Art $\Delta T_m > 6$ in qPCR+CE | 6-FAM (hexanyl) A+ATTCCCGTC GCTATCAA C3 | 18 | (2) (18) | LNA(+A) 3'-blocker C3 | 60,8 |
| 11 | Artificial | competitor Prior Art $\Delta T_m > 6$ in qPCR+CE | 6-FAM (CuAAC) C+GTCGCTATC AAGGAATTAA GAGAAGCA C3 | 28 | (2) (28) | LNA (+G) 3'-blocker C3 | 67,1 |
| 12 | Artificial | upstream probe Alternative b) in dPCR | 6-FAM T+AAAATTCCC GTCGCTATCA AGGA TQ2 | 24 | (2) (24) | LNA (+A) Quencher TQ2 | 65,2 |
| 13 | Artificial | downstream probe Alternative b) in dPCR | HEX C+GCTATCAAG GAA+TTAAGAG AAGCA TQ2 | 25 | (2) (14) (25) | LNA (+G) LNA (+T) Quencher TQ2 | 65,4 |
| 14 | Artificial | upstream probe Alternative a) in qPCR+CE | 6-FAM (hexanyl) T+AAAATTCCC GTCGCTATCA AGGA C3 | 24 | (2) (24) | LNA (+A) 3'-blocker C3 | 65,2 |
| 15 | Artificial | downstream probe Alternative a) in qPCR+CE | 6-FAM (CuAAC) C+GCTATCAAG GAA+TTAAGAG AAGCA C3 | 25 | (2) (14) (25) | LNA (+G) LNA (+T) 3'-blocker C3 | 65,4 |
| 16 | Artificial | upstream probe Alternative c) | 6-FAM (hexanyl) T+AAAATTCCC GTCGCTATCA AGGA TQ2 | 24 | (2) (24) | LNA (+A) Quencher TQ2 | 65,2 |

(continued)

| Seq ID No | Organism | Name | sequence (5' → 3'-end) | length | Loca-tion | Gene/Feature/ modification | $T_a$ [°C] |
|---|---|---|---|---|---|---|---|
| 17 | Artificial | downstream probe Alternative c) | Dy-631 C+GCTATCAAG GAA+TTAAGAG AAGCA TQ2 | 25 | (2) (14) (25) | LNA (+G) LNA (+T) TQ2 | 65,4 |
| 18 | Artificial | Primer F/FLT3 | TCACGGCACA GCCCAGTAAA | 20 | (1-20) | on Seq ID No. 22 | |
| 19 | Artificial | Primer R/FLT3 | AATAGCAGCC TCACATTGCC C | 21 | (250-271) | on Seq ID No. 22 | |
| 20 | Artificial | Primer F/NPM1 | ATGTCTATGAAGTGTTGGTTCCTTAAC | 29 | (1-29) | on Seq ID No. 23 | |
| 21 | Artificial | Primer R/NPM1 | ACACGGTAGGGAAAGTTCTCACTC | 24 | (181-205) | on Seq ID No. 23 | |
| 22 | Homo Sapiens | FLT3-T1-w | TCACGGCACA GCCCAGTAAA GATAAGAGGC CTTCCATCAC CGGTACCTCC TACTGAAGTT GAGTCTAGAA GAAAGATTGC ACTCCAGGAT AATACACATC ACAGTAAATA ACACTCTGGT GTCATTCTTG ACAGTGTGTT CACAGAGACC TGGCCGCCAG GAACGTGCTT GTCACCCACG GGAAAGTGGT GAAGATATGT GACTTTGGAT TGGCTCGAGA TATCATGAGT GATTCCAACT ATGTTGTTCAG GGGCAATGTG AGGCTGCTAT T | 271 | | FLT3, GRCh38, 13: 28018453..28018723 | N/A |
| 23 | Homo Sapiens | NPM1-T1-w | ATGTCTATGA AGTGTTGTGG TTCCTTAACCA CATTTCTTT TTTTTTTTT CCAGGCTATT CAAGATCTCT GGCAGTGGAG GAAGTCTCTT TAAGAAAATA GTTTAAACAA TTTGTTAAAA AATTTTCCGT CTTATTTCAT TTCTGTAACA GTTGATATCT GGCTGTCCTT TTTATAATGC AGAGTGAGAA CTTTCCCTAC CGTGT | 205 | | NPM1, GRCh38, 5: 171410473..17141067 7 | N/A |

36

**Standard polymerase chain reaction (PCR) setup and PCR cycling using the Bio-Rad's Droplet Digital PCR system:** The standard PCR reaction was set up in a final volume of 20 $\mu$L and contained one-fold ddPCR Supermix (Bio-Rad Laboratories, Inc., Hercules, California, USA), 6,200 copies of gBlock DNA, containing the target sequences, 0.2 $\mu$M PCR primers (Seq ID 6-7), and 0.2 $\mu$M probes (two out of Seq ID 8-15). For sample partitioning, the 20 $\mu$L PCR reaction was loaded together with 70 $\mu$L Droplet Generation Oil (Bio-Rad Laboratories, Inc., Hercules, California, USA) into a DG8 Cartridge (Bio-Rad Laboratories, Inc., Hercules, California, USA) and droplets were generated with the QX200 Droplet Generator. The droplets (40 $\mu$L) were transferred to a PCR plate and the plate was sealed using Bio-Rad's PX1 PCR Plate Sealer (Bio-Rad Laboratories, Inc., Hercules, California, USA) and pierceable heat seal. The PCR plate was transferred in a thermal cycler and run with the following thermal cycling protocol at a ramp rate of 2.5°C/sec: 10 min of hotstart activation at 95 °C, followed by 40 cycles of 30 s at 94 °C and 60 s at 62 °C and one cycle of 10 min at 98 °C. Following PCR amplification of the nucleic acid target in the droplets, the droplets were read out using the QX200 Droplet Reader (Bio-Rad Laboratories, Inc., Hercules, California, USA). The reader measures fluorescence intensity of each droplet and detects the size and shape as droplets pass the detector. All instruments were operated according to the manufacturer's instructions.

**Standard polymerase chain reaction (PCR) setup for the Modaplex® system:** Standard PCR was performed in a final volume of 25 $\mu$L and contained one-fold Modaplex Buffer 9 (Art. No. 85-20201, Biotype GmbH, Dresden, Germany), 2 units biotechrabbit Hot StartTaq DNA polymerase (Cat. No. BR0200102, biotechrabbit GmbH, Berlin, Germany), 6,200 copies of gBlock DNA, containing the target sequences, 0.2 $\mu$M PCR primers, 0.2 $\mu$M probes and a size standard. Multiwell plates were sealed with Aluminum Sealing Film (Biotype) and spun using a Bench-top centrifuge with plate adaptor. Thereafter the sealing was gently removed and 5 $\mu$L of mineral oil (Merck - SIGMA-Aldrich, Darmstadt, Germany) were applied to each well.

**PCR cycling combined with capillary electrophoresis using the Modaplex® system:** The analyzer is a modular device consisting of a PCR thermocycler, an automatic sampler, a capillary gel electrophoresis device and a fluorescence detector with two analysis channels (US7445893, US7081339, US7674582, US8182995; Hlousek et al. 2012). The analyzer was completely operated with reagents of the manufacturer and according to its instructions. The PCR was set up with the chemicals of the manufacturer, wherein the final concentrations of PCR primers and probes corresponded to those described by standard PCR. The PCR program comprised of 2 min of hotstart activation at 96 °C, followed by 16 cycles of 45 s at 62 °C, 45 s at 72 °C and 5 s at 98 °C, and 28 cycles, including 12 injections for CE separation, of 45 s at 62 °C, 220 s at 72 °C and 10 s at 96 °C. Real time analysis by capillary gel electrophoresis was carried out 12 times in the elongation phase at 72 °C by electrokinetic injection (10,000 V, 15 s) from 17th to 39th cycle (every second cycle). For the Bio-Rad oligo design the 62° C temperature steps were shifted to 55 °C, as this corresponds to the optimal annealing temperature of the oligo designs. The detection unit records fluorescence, expressed in relative fluorescence units (RFU), and provides an electropherogram as an output (see Fig. 3 and 4). Non-amplifying internal calibrators, present on both analysis channel, are used in each assay to calculate migration lengths of hydrolysis fragments.

**Example 1: Comparison of probe design alternative b) and probe design of prior Art (PA) using Droplet Digital PCR for variant detection**

This example describes exemplary digital amplification assays for detecting the presence of a mutation in exon 19 of the epidermal growth factor receptor (EGFR), see Figure 2 & 3. Standard PCR was set up for the target Seq ID No. 1 (T1-w), Seq ID No. 2 (T1-v/SNP) , Seq ID No. 3 (T1-v/DEL1), Seq ID No. 4 (T1-v/DEL2) , Seq ID No. 5 (T1-v/DEL9) with the primers Seq ID No. 6 and Seq ID No. 7 and the probes (Seq ID No. 8 (probe) Seq ID No. 9 (competitor)) for a design according to the prior art and the probes (Seq ID No. 12 (upstream probe), Seq ID No. 13 (downstream probe)) for the inventive probe design. The competitor and probe may be labeled with different fluorophores. For the data presented in this Example, the competitor is labeled at its 5' terminus with a HEX fluorescent dye, and the probe is labeled at the 5' terminus with a FAM fluorescent dye, with each dye conjugated to the 5' terminal nucleotide of the corresponding oligonucleotide. Each of the competitor and probe also is conjugated to a TQ2 quencher at the 3' terminus.

The FAM and HEX signal values (in arbitrary units (arb.)) obtained for individual droplets after PCR were graphed to produce the scatter plots of Fig. 1 and 2, where the two signal values (FAM and HEX) for each droplet are represented by the position of a dot in the plot. Fig. 1 shows an overlay of plots of droplet signal values detected from droplets containing 100 % wild-type (A, D), 100 % variation (T1-v) (B; E) or 0.5 % variation (T1-v) with 99.5 % wild-type form (C; F) of an exon 19 EGFR target, after amplification of the target in the droplets for both the Bio-Rad oligo design and the inventive probe design. Clusters of droplets containing neither form of the target (quadrant III), only the variation form ("variation", quadrant I), and only the wild-type form ("wild-type", quadrant IV) are identified in the plot. Alt. b) probe design (Panel D, E, F) allowed for a much clearer separation of variation and wild-type clusters in quadrant IV and I, respectively, than the design according to the prior art (Panel A, B, C). Both designs allow for the detection of 0.5 % mutant DNA in 99.5 % wild-type DNA, however, the probe design b) also allows for the detection of 0.1 % mutant DNA in 99.9 % for all mutant variant tested. In contrast the design according to the prior art only achieved this sensitivity for large scale deletions. Figure 3 illustrates representative results obtained from targets with low variation frequency, that for example occur in samples analyzing the measurable residual disease (MRD). Individual target variations are diluted in the wildtype T1-w at a frequency of 0,5 % and plotted for both the probe design according to the teachings of the prior art and the probe design according to the present invention. In the case of a small 1nt deletion (upper right panels), the probe design according to the present invention clearly delineates individual droplets in quadrant 1, while the cluster in quadrant IV of the probe design according to the prior art is not distinguishable from the wildtype fraction.

**Example 2: Alt. a) probe design based on inventive design rules with or without a LNA on the Modaplex instrument**

Standard Modaplex PCR protocol was set up for the design according to the prior art for the target Seq ID No. 1 (T1-w) and Seq ID No. 5 (T1-v/DEL9) with the primers Seq ID No. 6 and Seq ID No. 7 and the probes with (Seq ID No. 10 (probe), Seq ID No. 11 (competitor)) or without a LNA (Seq ID No. 10 & 11 without the feature at nucleotide (2) (five prime blocker) but with a 5'-6-FAM and a 3'-Spacer C3 modification. The cleavage of probes without the LNA revealed several peaks in Modaplex-CE (Fig. 4, secondary hydrolysis peaks highlighted with an asterisk). This phenomenon makes the system unusable because there are many signal peaks per target (reduced multiplexing capability) and, additionally, the hydrolysis pattern is probe dependent (peak strength varies with probe which suggests probes are hydrolyzed differently based on their sequence). To circumvent this problem, the nucleotide analog "locked nucleic acid" (LNA), which (i) delays nuclease activity and (ii) increases annealing temperature (Owczarzy et al. 2011), was inserted at the $2^{nd}$ position from the 5' end based on the inventive design rules. Cleavage of the alt. a) probes of the present invention now resulted in a defined and distinct signal peak per target (see Fig. 4). The here observed residual side peaks can be further reduced by blocking with an additional LNA at position 2 from the 5' end.

**Example 3: Comparison of the probe design according to prior art and alt. a) probe design using the Modaplex system for variant detection**

This example describes exemplary Modaplex amplification assays for detecting the presence of a mutation in exon 19 of the epidermal growth factor receptor (EGFR), see Fig. 5. Standard PCR was set up for the target without variation Seq ID No. 1 (T1-w) and the target with variation Seq ID No. 5 (T1-v/DEL9) with the primers Seq ID No. 6 and Seq ID No. 7 and the probes (Seq ID No. 10 (probe) Seq ID No. 11 (competitor)) for a design according to the prior art and the probes (Seq ID No. 14 (upstream probe), Seq ID No. 15 (downstream probe)) for the inventive probe design with a 5'-6-FAM, a 3'-Spacer C3 and internal LNA modifications.
The upstream probe and the downstream probe can be labeled with identical fluorophores. For the data presented in this Example, the probes were labeled at their 5' terminus with a 6-FAM fluorescent dye, a Spacer C3 at their 3' terminus and a LNA at the $2^{nd}$ position from the 5' end, to avoid hydrolysis peaks aside from the 1-nt hydrolysis peak.
The FAM signal (in arbitrary units (arb.)) detected after PCR and capillary electrophoresis is shown as electropherograms in Fig. 4, 5 and 7 at 100 % wild-type, 100 % variation and 5 % variation in 95 % wild-type form of an exon 19 EGFR target. The peaks SST1 and SST2 correspond to internal calibrators that are used to calculate migration lengths of hydrolysis fragments. The peaks for the upstream/downstream probe according to Alt. a) and probe/competitor according to prior art are indicated. The preliminary Limit of Detection (pLOD) obtained for different mutant variants are shown in Table 3. The probe of the present invention allowed for a pLOD of 5-10 % depending on the variation, while the design according to prior art only allowed for a pLOD of 10-100%.

**Table 3: Preliminary Limit of Detection (pLOD).**

| Method | ddPCR | | qPCR + CE | |
|---|---|---|---|---|
| Design according to | Prior Art | Alt. b) | Prior Art | Alt. a) |
| 9bp deletion | 0,5% | 0,5% | 50% | 5% |
| 2bp deletion | 0,5% | 0,5% | 50% | 5% |
| 1bp deletion | 100% | 0,5% | 50% | 5% |
| SNP | 0,5% | 0,1% | 50% | 5% |

The pLOD results are obtained by analyzing mixed samples of individual targets with variation in wildtype for two methods and probe designs compared between teachings of the prior art and the present invention. In conclusion, the probe design according to the teachings of the present inventions result in more sensitive tests regarding the Limit of Detection. Using the Alt. b) enables the detection of variations even with very low variation frequency as it can be expected in MRD samples. Especially a 1nt deletion cannot be reliably distinguished from wildtype with probes designed according to the teachings of prior art. Furthermore, probes of the prior art are not suitable for detection of mutations in bulk reaction such as qPCR + CE. The slightly lower sensitivity of Alt. a) in comparison to Alt. b) will still be useful for the simultaneous multiplex detection of several different variations sites in one reaction, which can be applied in cancer diagnostic.

**Example 4**

First applications in AML diagnostics enable results about FLT3 TKD and NPM1 status in one well for each patient sample in less than four hours. The FLT3 TKD and the NPM1 status is analyzed by means of the probe design alternative a) (Fig. 7). Standard PCR was set up for the targets FLT3 (Seq ID No. 22) and NPM1 (Seq ID No. 23) at commonly known mutation sites relevant to AML diagnostics (Döhner et al., 2022) with the primers Seq ID No. 18 and Seq ID No. 19 for FLT3 and the primers Seq ID No. 20 and Seq ID No. 21 for NPM1. In addition, this application contains primer-specific detection of IDH1 and IDH2 mutations as well as amplicon-length based detection of FLT3 ITD insertions in the same multiplex. Thus, the herein described multiplex enables concomitant detection of analyte length variations (FLT3 TKD), SNP (IDH1, IDH2, FLT3 TKD) and InDel Mutations ≤4 bp (NPM1, FLT3 TKD) and is feasible for combination with other desired biomarkers (table 4).

**Table 4 AML Biomarker panel**

| Biomarker /gene | gene | COSMIC ID* | Type of variation | Genomic coordinates ( GRCh38) |
|---|---|---|---|---|
| FLT3 | fms-like tyrosine kinase 3 (Tyrosine Kinase Domain) | COSM783 COSM784 COSM785 COSM796 COSM797 | dbSNP; Pos. 2503, G>T Subsitition, Pos. 2504, A>T Subsitition; Pos. 2503, G>C Deletion; Pos. 2503-2505 Deletion; Pos. 2508-2510 | 13:28018505..28018505 13:28018504..28018504 13:28018505..28018505 13:28018503..28018505 13:28018500..28018502 |
| NPM1 | Nucleo phosmin 1 | COSM 17559 COSM 17571 COSM 17572 COSM 17573 | 860_863insTCTG (Insertion) 863_864insCATG (Insertion) 863_864insCGTG (Insertion) 863_864insCCTG (Insertion) | 5:171410543.. 171410544 |
| * COSMIC ID means Legacy mutation identifier (COSM) which represents existing COSM mutation identifiers. This identifier remains the same between different assemblies (GRCh37 and GRCh38). All the COSM ids at the same genomic location have been collapsed into one representative COSM id. These ids are maintained to help track existing mutations. | | | | |

The detected variants of example 4 represent common AML associated variants and are by no means a complete list of variants that may be detected using the method according to the invention. Biomarker representing other common AML variants are summarized in Döhner et al 2022. Starting from page 1355 Döhner et al discusses different technologies for the diagnosis of AML such as multiparameter flow cytometry-based MRD (MFC-MRD) and molecular MRD (Mol-MRD) assessed by qPCR, next-generation sequencing (NGS) and dPCR. With referencing to table 7 Döhner et al criticizes that real-time quantitative PCR (RT-qPCR) would have limited applicability for targets such as NPM1, CBFB::MYH11, RUNX1::RUNX1T1, KMT2A::MLLT3, DEK::NUP214 and BCR::ABL1, WT1. However, the method of the present invention provides a new teaching for the expansion of the qPCR applicability for AML diagnosis. The present example shows that multiplexing tailored for AML diagnosis enables the detection of each desired variant. The use of the CE device, such as Modaplex, allows to increase the multiplex grade. The same applies for other variants used as biomarker and other leukemia comprising CML, AML, ALL, CLL, MDS, MPN.

Other relevant AML biomarkers are disclosed in Döhner et al 2022 (e.g. CEBPA,#DDX41, TP53; ASXL1, BCOR, EZH2, RUNX1, SF3B1, SRSF2, STAG2, U2AF1, ZRSR2, ANKRD26, BCORL1, BRAF, CBL, CSF3R, DNMT3A, ETV6, GATA2, JAK2, KIT, KRAS, NRAS, NF1, PHF6, PPM1D, PTPN11, RAD21, SETBP1, TET2, WT1). If desired those can be combined for any AML diagnostic for use in the method and with a probe design a), b) and/or c) according to the present invention.

As shown in example 2, similar results for sensitivity were obtained for NPM1 with 0.1% degree of variation in singleplex and 1% degree of variation in multiplex. Regarding the state of the art, a sensitivity of -0.1% as standard for MRD/chimerism analysis is required: This is performed after transplantation of a patient and when the respective Mutations are known. Therefore, the singleplex method of the present invention can be used to achieve the same sensitivity in single plex reactions on a variety of devices such as for digital PCR or NGS; preferably by means of the probe design alternative b) and/or c). The maximum sensitivity of the probes and the method can be improved by modifications, e.g. with internal nuclease blocker, e.g. PTO, LNA-modifications or FEN-enhancer. A 1% sensitivity in the initial diagnostic application is comparable to prior art FISH assays. Thus, the present invention, at least shows suitable sensitivity for clinical samples even in Multiplex settings. Even more importantly, with respect to polymorphism (VAR) detection and quantification, the present invention offers high potential to address these mutations in Multiplex approaches without the downsides of allele-specific primers or probes. While allele-specific primer or probes require detailed information about each polymorphism in a hotspot region, the probes of the present invention can be designed based on the wildtype sequence. The present invention can discriminate between wildtype and all possible variants of polymorphisms in a hotspot without disturbing primer interactions or limitations in the number of variations detected.

## Claims

1. A method for the detection of at least one or more molecular genetic analytes comprising

   - providing a reaction mixture comprising

      • at least one molecular genetic analyte comprising at least one target sequence (T1) without a variation (T1-w) and potentially at least one molecular genetic analyte comprising at least one target sequence with at least one variation (T1-v),
      • a primer pair (F, R) flanking the at least one T1-w and the at least one T1-v being suitable for non-discriminatory amplification of the target sequences,
      • at least one **upstream oligonucleotide** probe ("upstream probe") comprising

         (i) a sequence region (1) and a sequence region (2), which together compose a sequence which is complementary to a sequence that is the same on the target sequence without (T1-w) and on the target sequence with at least one variation (T1-v), and

      • at least one competitive **downstream oligonucleotide** probe ("downstream probe") comprising

         (i) a sequence region (3) and a sequence region (2), which together compose a sequence which is complementary to a sequence on the target sequence without a variation (T1-w), and
         (ii) at least one mismatch within the region (3) in relation to the at least one variation on the target sequence (T1-v),

      • the 3'-end of the upstream and 5'-end of the downstream probe are complementary to the partially overlapping region (2) on a sequence 3' upstream of the at least one variation on the at least one target sequence (T1-v), and
      • the sequence region (1) and the sequence region (3) have a similar melting temperature (Tm) and a $\Delta$ Tm sequence region (1) to Tm sequence region (3) of less than or equal to 6°C,
      wherein **each** probe further comprises:

         (iii) a protective group (3'-blocker) at the 3'-end of probe inhibiting a primer extension reaction, and
         (iv) at least one cleavable hydrolysis product from the 5'-end of the respective probe (F1-Down and/or F1-up) comprising at least one nucleotide of the 5'-end of the respective probe, and

• the at least one downstream probe and/or the upstream probe comprises (v) a label at 5'-end,

the method comprising the steps
- contacting the at least one target sequence with the at least one downstream probe and the least one upstream probe and the at least one primer pair (F, R),
- hybridization of the at least one primer pair (F, R)
- hybridization of at least one probe to its respective complementary sequence on the at least one target sequence, wherein the respective probe hybridizes within the region flanked by the primer pair (F, R), and

• the upstream probe and the downstream probe hybridize in equilibrium to the at least one target sequence without a variation (T1-w),
• the downstream probe hybridizes with a decreasing hybridization rate in relation to the upstream probe to the at least one target sequence with the at least one variation (T1-v), and
• the upstream probe hybridizes with an increased hybridization rate in relation to the downstream probe to the at least one target sequence with the at least one variation (T1-v),

- cleavage of the hybridized probe(s) with a target-dependent 5' to 3' nuclease activity to release the at least one cleavable hydrolysis product (F1-Up and/or F1-Down), and
- detection of the achieved hydrolysis product(s) (F1-Up and/or F1-Down).

2. The method of claim 1, wherein with increasing $\Delta$ Tm of Tm sequence region (3) to Tm of the respective sequence on T1-v comprising the at least one analyte, the hybridization rate of the downstream probe to T1-v decreases.

3. The method of claim 1 or 2, wherein the increase or decrease of the hybridization rate is mediated by a competitive hybridization of the partially overlapping regions (2) of the at least one downstream and the at least one upstream probe to the respective complementary sequence.

4. The method according to any one of claims 1 to 3, wherein the method further comprises a non-discriminatory amplification of the respective sequence.

5. The method according to any one of the claims 1 to 4, wherein

- according to an alternative a) the at least one upstream probe and the at least one downstream probe comprises respectively (iii) a protective group (3'-blocker) at the 3'-end of probe inhibiting a primer extension reaction and (iv) at least one cleavable hydrolysis product from the 3'-end of the respective probe comprising at least one internal nuclease blocker at the 5'-region of the hybridizing sequence of the probe conferring resistance to a nuclease activity and structurally dividing a cleavable hydrolysis product from the 3'-end of the respective probe which comprises at least one nucleotide, and (v) at least one label bridged via a linker to the 5'-end of cleavable hydrolysis product of at least one probe or
- according to an alternative b) the upstream probe and the downstream probe comprises (iii) a protective group (3'-blocker) at the 3'-end of the probe inhibiting a primer extension reaction and which has a quencher function and (v) the at least one upstream probe and the at least one downstream probe comprises a label wherein the labels in relation to each other are different or
- according to an universal alternative c) the upstream probe and the downstream probe comprises (iii) a protective group (3'-blocker) at the 3'-end of the probe inhibiting a primer extension reaction and which has a quencher function, the at least one upstream probe and the at least one downstream probe comprise (iv) at least one cleavable hydrolysis product from the 3'-end of the probe comprising at least one internal nuclease blocker at the 5'-region of the hybridizing sequence of the downstream probe conferring resistance to a nuclease activity and structurally dividing a cleavable hydrolysis product from the 3'-end of the downstream probe which comprises at least one nucleotide, and (v) the at least one upstream probe and the at least one downstream probe comprises a label wherein the labels in relation to each other are different.

6. The method according to any one of the claims 1 to 5, wherein the method further comprise a step of

- calculation of the ratio between the upstream and downstream hydrolysis product(s) (F1-Up, F1-Down) on the target without variation (T1-w), the same ratio between the upstream and downstream hydrolysis product(s) (F1-Up, F1-Down) on the target with the at least one variation (T1-v) and comparison of the ratios (F1-Up, F1-Down [T1-w] and F1-Up, F1-Down [T1-v]) representing the degree of variation of the at least one molecular genetic analyte.

7. The method according to any one of the claims 1 to 6, wherein in alternative a) a non-discriminatory amplification of the respective sequence by means of a real-time PCR and a step of separation of the achieved hydrolysis products (F1-Up and/or F1-Down) are performed.

8. The method according to any one of the claims 1 to 6, wherein in alternative b) a step of partitioning of the reaction mixture and a non-discriminatory amplification by means of an end point PCR are performed.

9. The method according to any one of the claims 1 to 7, wherein at least one internal nuclease blocker is located in region (2) of downstream probe and at least one internal nuclease blocker in region (1) of the upstream probe.

10. The method of any one of the claims 1 to 9, wherein variation within the at least one target sequence with the at least one variation (T1-v), comprises

- at least one deletion of at least one base pair,
- at least one insertion of at least one base pair,
- at least one deletion-insertion polymorphism (DIP), and/or
- at least one single nucleotide polymorphism (SNP).

11. The method according to any one of claims 1 to 10, wherein a plurality of downstream probes and a respective plurality of upstream probes are used to detect a plurality of analytes.

12. A combination of at least two oligonucleotide probes ("probe") comprising

• at least one **upstream oligonucleotide** probe ("upstream probe") comprising

(i) a sequence region (1) and a sequence region (2), which together compose a sequence which is complementary to a sequence that is the same on the target sequence without (T1-w) and on the target sequence with at least one variation (T1-v), and

• at least one competitive **downstream oligonucleotide** probe ("downstream probe") comprising

(i) a sequence region (3) and a sequence region (2), which together compose a sequence which is complementary to a sequence on the target sequence without a variation (T1-w), and
(ii) at least one mismatch within the region (3) in relation to the at least one variation on the target sequence (T1-v),

• the 3'-end of the upstream and 5'-end of the downstream probe are complementary to the partially overlapping region (2) on a sequence 3' upstream of the at least one variation on the at least one target sequence (T1-v), and
• the sequence region (1) and the sequence region (3) have a similar melting temperature (Tm) and a $\Delta$ Tm sequence region (1) to Tm sequence region (3) of less than or equal to 6°C, wherein

- according to an alternative a) the at least one upstream probe and the at least one downstream probe comprises respectively (iii) a protective group (3'-blocker) at the 3'-end of probe inhibiting a primer extension reaction and (iv) at least one cleavable hydrolysis product from the 3'-end of the respective probe comprising at least one internal nuclease blocker at the 5'-region of the hybridizing sequence of the probe conferring resistance to a nuclease activity and structurally dividing a cleavable hydrolysis product from the 3'-end of the respective probe which comprises at least one nucleotide, and (v) at least one label bridged via a linker to the 5'-end of cleavable hydrolysis product of at least one probe or
- according to an alternative b) the upstream probe and the downstream probe comprises (iii) a protective group (3'-blocker) at the 3'-end of the probe inhibiting a primer extension reaction and which has a quencher function (iv) at least one cleavable hydrolysis product from the 3'-end of the respective probe (F1-Down and/or F1-up) comprising at least one nucleotide of the 5'-end of the respective probe and (v) the at least one upstream probe and the at least one downstream probe comprises a label wherein the labels in relation to each other are different or
- according to an universal alternative c) the upstream probe and the downstream probe comprises (iii) a protective group (3'-blocker) at the 3'-end of the probe inhibiting a primer extension reaction and which has a quencher function, the at least one upstream probe and the at least one downstream probe comprise (iv) at least one cleavable hydrolysis product from the 3'-end of the probe comprising at least one internal nuclease blocker at the 5'-region of the hybridizing sequence of the downstream probe conferring resistance to a nuclease activity and structurally dividing a cleavable hydrolysis product from the 3'-end of the downstream probe which comprises at least one nucleotide, and (v) the at least one upstream probe and the at least one downstream probe comprises a label wherein the labels in relation to each other are different.

13. At least one hydrolysis product released from the respective upstream probe (F1-Up) and at least one hydrolysis product released from downstream probe (F1-Down) according to any one of the claims 1 to 10.

14. Use of the combination of at least one downstream probe and of the least one upstream probe according to claim 12 and/or of the respective hydrolysis product according to claim 13 in a method for the detection of at least one or more analytes according to any one of the claims 1 to 11.

15. The use according to claim 14, wherein the released hydrolysis product, preferably of the labeled released hydrolysis product, respectively is the confirmation of the presence of at least one analyte.

16. At least one labeled amplicon of A1-v and at least one labeled amplicon A1-w, for alternative a) in comparison being the confirmation of a specific variation of the at least one analyte.

17. A kit comprising

- the combination of probes according to claim 12
- at least one primer pair (R, F) specific for the respective target to be amplified and
- optionally a reference representing T1-w enabling detection of the at least one molecular genetic analyte comprising a target sequence with at least one variation (T1-v).

Fig. 1

Fig. 2

A    **probe design PA ΔTm>6**

D    **probe design Alternative b)**

B    **100% Variation (overlay)**

E    **100% Variation (overlay)**

C  **0.5% Variation + 95.5% WT (overlay)**

F  **0.5% Variation + 95.5% WT (overlay**

Fig. 3

Fig. 4

Fig. 5

Fig. 6

```
SEQ ID 1 T1-w          CCCAGAAGGTGAGAAAGTTAAAATTCCCGTCGCTATCAAGGAATTAAGAGAAGCAACATCTCCGAAACCCAACAAGGAAA
         T1-v          |||||||||||||||||||||||||||||||||||||||||||||       |||||||||||||||||||||||||||||
SEQ ID 2 SNP           CCCAGAAGGTGAGAAAGTTAAAATTCCCGTCGCTATCAAGGAAGTAAGAGAAGCAACATCTCCGAAACCCAACAAGGAAA
SEQ ID 3 1nt del.      CCCAGAAGGTGAGAAAGTTAAAATTCCCGTCGCTATCAAGGA-TTAAGAGAAGCAACATCTCCGAAACCCAACAAGGAAA
SEQ ID 4 2nt del.      CCCAGAAGGTGAGAAAGTTAAAATTCCCGTCGCTATCAAGGA--TAAGAAGCAACATCTCCGAAACCCAACAAGGAAA
SEQ ID 5 9nt del.      CCCAGAAGGTGAGAAAGTTAAAATTCCCGTCGCTATCAAGGA----GC-----CAACATCTCCGAAACCCAACAAGGAAA
                                                          ||||||||||||          ||
SEQ ID 15 Alt.a) downstream p.       6-Fam/CuAAC  CGCTATCAAGGAATTAAGAGAAGCA C3
SEQ ID 14 Alt.a) upstream p.    6-Fam/C6 TAAAATTCCCGTCGCTATCAAGGA C3

SEQ ID 13 Alt.b) downstream p.              Hex CGCTATCAAGGAATTAAGAGAAGCA TQ2
SEQ ID 12 Alt.b) upstream p.          6-Fam TAAAATTCCCGTCGCTATCAAGGA TQ2

SEQ ID 17 Alt.c) downstream p.           Dy-631 CGCTATCAAGGAATTAAGAGAAGCA TQ2
SEQ ID 16 Alt.c) upstream p.     6-Fam/C6 TAAAATTCCCGTCGCTATCAAGGA TQ2
```

Fig. 7

Europäisches
Patentamt

European
Patent Office

Office européen
des brevets

# EUROPEAN SEARCH REPORT

Application Number

EP 22 20 0403

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2009/073251 A1 (SEQUENOM INC [US]; VAN DEN BOOM DIRK JOHANNES [US] ET AL.) 11 June 2009 (2009-06-11) <br> * abstract * <br> * figures 2,4 * <br> * page 41, line 25 - line 28 * <br> * example 4 * <br> * claim 16 * | 12,13, 16,17 | INV. <br> C12Q1/6823 <br> C12Q1/6827 <br> C12Q1/6851 |
| X | WO 2016/041591 A1 (ROCHE DIAGNOSTICS GMBH [DE]; HOFFMANN LA ROCHE [CH] ET AL.) 24 March 2016 (2016-03-24) <br> * abstract * <br> * page 19, line 14 - line 22; example 3 * <br> * example 3 * <br> * claims 2,5,10 * <br> * figures 3-5 * | 12,13, 16,17 | |
| A,D | WO 2017/046191 A1 (BIOTYPE DIAGNOSTIC GMBH [DE]) 23 March 2017 (2017-03-23) <br> * abstract * <br> * claims 1,6,8,20,22 * <br> * figure 1 * | 1-17 | TECHNICAL FIELDS SEARCHED (IPC) <br><br> C12Q |
| A | EP 3 739 064 A1 (BIOTYPE GMBH [DE]) 18 November 2020 (2020-11-18) <br> * abstract * <br> * claims 1,12-14 * <br> * page 3; figure 3 * | 1-17 | |

-/--

The present search report has been drawn up for all claims

| Place of search | Date of completion of the search | Examiner |
|---|---|---|
| Munich | 20 March 2023 | Barz, Wolfgang |

EPO FORM 1503 03.82 (P04C01)

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | KEIGHTLEY MARIA CRISTINA ET AL: "Real-time NASBA detection of SARS-associated coronavirus and comparison with real-time reverse transcription-PCR", JOURNAL OF MEDICAL VIROLOGY, vol. 77, no. 4, 1 January 2005 (2005-01-01), pages 602-608, XP055823329, US ISSN: 0146-6615, DOI: 10.1002/jmv.20498 * abstract * * page 604 * * figures 1-2 * * discussion * | 1-17 | |

-----

**TECHNICAL FIELDS SEARCHED (IPC)**

The present search report has been drawn up for all claims

| Place of search | Date of completion of the search | Examiner |
|---|---|---|
| Munich | 20 March 2023 | Barz, Wolfgang |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons
..........................................................................
& : member of the same patent family, corresponding
   document

EPO FORM 1503 03.82 (P04C01)

**ANNEX TO THE EUROPEAN SEARCH REPORT
ON EUROPEAN PATENT APPLICATION NO.**

EP 22 20 0403

This annex lists the patent family members relating to the patent documents cited in the above-mentioned European search report.
The members are as contained in the European Patent Office EDP file on
The European Patent Office is in no way liable for these particulars which are merely given for the purpose of information.

20-03-2023

| Patent document cited in search report | | Publication date | Patent family member(s) | | Publication date |
|---|---|---|---|---|---|
| WO 2009073251 | A1 | 11-06-2009 | US 2008305479 A1 | | 11-12-2008 |
| | | | WO 2009073251 A1 | | 11-06-2009 |
| WO 2016041591 | A1 | 24-03-2016 | CA 2961417 A1 | | 24-03-2016 |
| | | | CN 106687597 A | | 17-05-2017 |
| | | | EP 3194613 A1 | | 26-07-2017 |
| | | | ES 2718510 T3 | | 02-07-2019 |
| | | | JP 6649367 B2 | | 19-02-2020 |
| | | | JP 2017527298 A | | 21-09-2017 |
| | | | WO 2016041591 A1 | | 24-03-2016 |
| WO 2017046191 | A1 | 23-03-2017 | DE 102015115836 A1 | | 23-03-2017 |
| | | | EP 3350340 A1 | | 25-07-2018 |
| | | | ES 2877154 T3 | | 16-11-2021 |
| | | | US 2018363040 A1 | | 20-12-2018 |
| | | | WO 2017046191 A1 | | 23-03-2017 |
| EP 3739064 | A1 | 18-11-2020 | CN 113891943 A | | 04-01-2022 |
| | | | EP 3739064 A1 | | 18-11-2020 |
| | | | EP 3969615 A1 | | 23-03-2022 |
| | | | US 2022205025 A1 | | 30-06-2022 |
| | | | WO 2020229461 A1 | | 19-11-2020 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 1996032496 A2
- WO 2017046191 A1
- EP 0506889 B1
- EP 0632134 B1
- EP 1152062 B1
- WO 2014023318 A1
- US 7445893 B
- US 7081339 B
- US 7674582 B
- US 8182995 B

### Non-patent literature cited in the description

- **DÖHNER et al.** Diagnosis and Management of AML in Adults: 2022 ELN Recommendations from an International Expert Panel. *Blood,* 2022, vol. 140 (12), 1345-1377, https://doi.org/10.1182/blood.2022016867
- **HE ; SEELA.** Propynyl groups in duplex DNA: stability of base pairs incorporating 7-substituted 8-aza-7-deazapurines or 5-substituted pyrimidines. *Nucleic Acids Research,* 15 December 2002, vol. 30 (24), 5485-5496
- **HLOUSEK et al.** Automated high multiplex qPCR platform for simultaneous detection and quantification of multiple nucleic acid targets. *Biotechniques,* 2012, vol. 52 (5), 316-24
- Invitrogen-Molecular Probes. The Molecular Probes® handbook. A guide to fluorescent probes and labeling technologies. Thermo Fisher Scientific Inc, 2010
- **KEIGHTLEY et al.** Real-time NASBA detection of SARS-associated coronavirus and comparison with real-time reverse transcription-PCR. *J Med Virol.,* 2005, vol. 77 (4), 602-8
- **LYAMICHEV et al.** Structure-specific endonucleolytic cleavage of nucleic acids by eubacterial DNA polymerases. *Science,* 1993, vol. 260 (5109), 778-83
- **MITSUNOBU et al.** Flap FLAP endonuclease activity of gene 6 exonuclease of bacteriophage T7. *J Biol Chem,* 2014, vol. 289, 5860-75
- **MCTGUE et al.** Sequence-Dependent Thermodynamic Parameters for Locked Nucleic Acid (LNA)-DNA Duplex Formation. *Biochemistry,* 2004, vol. 43 (18), 5388-5405
- **NATSUME et al.** Effect of base mismatch on the electronic properties of DNA-DNA and LNA-DNA double strands: density-functional theoretical calculations. *Chem. Phys. Lett.,* 2007, vol. 446, 151-158
- **OWCZARZY et al.** Stability and mismatch discrimination of locked nucleic acid-DNA duplexes. *Biochemistry,* 2011, vol. 50, 9352-9367
- **SANTALUCIA et al.** A unified view of polymer, dumbbell, and oligonucleotide DNA nearest-neighbor thermodynamics. *Proc. Natl. Acad. Sci. USA,* 1998, vol. 95 (4), 1460-1465
- **TAN et al.** Current commercial dPCR platforms: technology and market review. *Crit Rev Biotechnol.,* 15 March 2022, 1-32